# EUROPÄISCHE PATENTSCHRIFT

(11) **EP 2 102 348 B1**
(45) Veröffentlichungstag und Bekanntmachung des Hinweises auf die Patenterteilung: **21.11.2012**
(21) Anmeldenummer: 07847018.4
(22) Anmeldetag: 07.12.2007
(51) Int. Cl.: C12N 15/81, C07K 14/39, C12P 7/06

(54) **NEUER SPEZIFISCHER ARABINOSE-TRANSPORTER AUS DER HEFE PICHIA STIPITIS UND DESSEN VERWENDUNGEN**
NOVEL SPECIFIC ARABINOSE TRANSPORTER FROM THE YEAST PICHIA STIPITIS, AND USES THEREOF
NOUVEAU TRANSPORTEUR D'ARABINOSE SPÉCIFIQUE ISSU DE LA LEVURE PICHIA STIPITIS ET SES UTILISATIONS

(30) Priorität: 20.12.2006 DE 102006060381
(43) Veröffentlichungstag der Anmeldung: 23.09.2009
(73) Patentinhaber: Butalco GmbH, 6331 Hünenberg/Zug (CH)
(72) Erfinder: BOLES, Eckhard, 63303 Dreieich (DE); KELLER, Marco, 60437 Frankfurt (DE)
(74) Vertreter: Engelhard, Markus
(86) Internationale Anmeldenummer: PCT/EP2007/010668
(87) Internationale Veröffentlichungsnummer: WO 2008/080505

(56) Entgegenhaltungen:
- WO-A-2007/143245
- WO-A-2007/143247
- JEFFRIES T W ET AL: "Metabolic engineering for improved fermentation of pentoses by yeasts" APPLIED MICROBIOLOGY AND BIOTECHNOLOGY, SPRINGER VERLAG, BERLIN, DE, Bd. 63, 1. Januar 2004 (2004-01-01), Seiten 495-509, XP002999147 ISSN: 0175-7598
- RICHARD P ET AL: "Production of ethanol from L-arabinose by Saccharomyces cerevisiae containing a fungal L-arabinose pathway" FEMS YEAST RESEARCH, ELSEVIER SCIENCE, TOKYO, NL, Bd. 3, 1. Januar 2003 (2003-01-01), Seiten 185-189, XP002999146 ISSN: 1567-1356
- JEFFRIES THOMAS W ET AL: "Genome sequence of the lignocellulose-bioconverting and xylose-fermenting yeast Pichia stipitis" NATURE BIOTECHNOLOGY, Bd. 25, Nr. 3, März 2007 (2007-03), Seiten 319-326, XP008091168 ISSN: 1087-0156
- FONSECA C ET AL: "L-Arabinose transport and catabolism in yeast" FEBS JOURNAL, BLACKWELL PUBLISHING, LONDON, GB, Bd. 274, 1. Januar 2007 (2007-01-01), Seiten 3589-3600, XP007903587 ISSN: 1742-464X

### SEQUENCE LISTING

<110> Johann Wolfgang Goethe-Universität
<120> Neuer spezifischer Arabinose-Transporter aus der Hefe Pichia stipitis und dessen Verwendungen
<130> FB21618
<160> 4
<170> PatentIn version 3.2
<210> 1
   <211> 542
   <212> PRT
   <213> Pichia stipitis
<400> 1
   Met Ser Tyr Glu Asp Lys Leu Val Gln Pro Ala Leu Lys Phe Arg Thr
   1               5                   10                  15
   Phe Leu Asp Arg Leu Pro Asn Ile Tyr Asn Val Tyr Ile Ile Ala Ser
               20                  25                  30
   Ile Ser Cys Ile Ser Gly Met Met Phe Gly Phe Asp Ile Ser Ser Met
           35                  40                  45
   Ser Ala Phe Ile Gly Glu Asp Asp Tyr Lys Asn Phe Phe Asn Asn Pro
       50                  55                  60
   Gly Ser Asp Ile Gln Gly Phe Ile Thr Ser Cys Met Ala Leu Gly Ser
   65                  70                  75                  80
   Phe Phe Gly Ser Ile Val Ser Ser Phe Ile Ser Glu Pro Phe Gly Arg
                   85                  90                  95
   Arg Ala Ser Leu Leu Leu Cys Ser Phe Phe Trp Met Val Gly Ala Ala
               100                 105                 110
   Val Gln Ser Ser Ser Gln Asn Arg Ala Gln Leu Met Ile Gly Arg Ile
           115                 120                 125
   Ile Ala Gly Phe Gly Val Gly Phe Gly Ser Ser Val Ala Pro Val Tyr
       130                 135                 140
   Gly Ser Glu Leu Ala Pro Arg Lys Ile Arg Gly Phe Val Gly Gly Ile
   145                 150                 155                 160
   Phe Gln Phe Cys Val Thr Leu Gly Ile Leu Ile Met Phe Tyr Ile Cys
                   165                 170                 175
   Tyr Gly Leu His Phe Ile Asn Gly Val Gly Ser Phe Arg Ile Ala Trp
   180 185 190
   Gly Leu Gln Ile Val Pro Gly Leu Val Leu Phe Val Gly Cys Phe Phe
   195 200 205
   Ile Pro Glu Ser Pro Arg Trp Leu Ala Lys His Gly Tyr Trp Asp Glu
   210 215 220
   Ala Glu Phe Ile Val Ala Gln Ile Gln Ala Lys Gly Asn Arg Glu Asp
   225 230 235 240
   Pro Asp Val Leu Ile Glu Ile Ser Glu Ile Lys Asp Gln Ile Leu Ile
   245 250 255
   Glu Glu Asn Leu Lys Ser Phe Gly Tyr Val Asp Leu Phe Thr Lys Lys
   260 265 270
   Tyr Ile Arg Arg Thr Leu Thr Ala Ile Phe Ala Gln Ile Trp Gln Gln
   275 280 285
   Leu Thr Gly Met Asn Val Met Met Tyr Tyr Ile Val Tyr Ile Phe Asn
   290 295 300
   Met Ala Gly Tyr Ser Asn Asn Ala Asn Leu Val Ala Ser Ser Ile Gln
   305 310 315 320
   Tyr Val Leu Asn Thr Ala Ala Thr Val Pro Ala Leu Phe Leu Met Asp
   325 330 335
   Tyr Ile Gly Arg Arg Arg Leu Leu Ile Gly Gly Ala Ile Met Met Met
   340 345 350
   Ile Phe Gln Phe Gly Val Ala Gly Ile Leu Gly Lys Tyr Ser Val Pro
   355 360 365
   Val Pro Gly Gly Leu Pro Gly Asn Pro Thr Val Thr Ile Gln Ile Pro
   370 375 380
   Glu Asp Asn Lys Ser Ala Ala Arg Gly Val Ile Ala Cys Cys Tyr Leu
   385 390 395 400
   Phe Val Val Ser Phe Ala Leu Ser Trp Gly Val Gly Ile Trp Val Tyr
   405 410 415
   Cys Ser Glu Val Trp Gly Asp Ser Ala Ser Arg Gln Arg Gly Ala Ala
   420 425 430
   Val Ser Thr Ala Ala Asn Trp Ile Leu Asn Phe Ala Ile Ala Met Tyr
           435                 440                     445
   Thr Pro Ser Ser Phe Lys Asn Ile Thr Trp Lys Thr Tyr Ile Ile Tyr
       450                 455                 460
   Ala Val Phe Cys Leu Val Met Ala Ile His Val Tyr Phe Gly Phe Pro
   465                 470                 475                     480
   Glu Thr Lys Gly Lys Arg Leu Glu Glu Val Gly Gln Met Trp Asp Glu
                   485                 490                     495
   Asn Val Pro Ala Trp Arg Ser Ser Ser Trp Gln Pro Thr Val Pro Leu
               500                 505                 510
   Leu Ser Asp Ala Asp Leu Ala His Lys Met Asp Val Ser His Lys Glu
           515                 520                 525
   Glu Gln Ser Pro Asp Ala Glu Ser Ser Ser Glu Glu Lys Pro
       530                 535                 540
<210> 2
   <211> 1629
   <212> DNA
   <213> Pichia stipitis
<400> 1
<210> 3
   <211> 1629
   <212> DNA
   <213> Pichia stipitis
<400> 3
   atgtcttacg aagacaagtt ggttcaacca gctttgaagt tcagaacctt cttggacaga     60
   ttgccaaaca tttacaacgt ttacattatt gcttctattt cttgtatttc tggtatgatg    120
   ttcggtttcg acatttcttc tatgtctgct ttcattggtg aagacgacta caagaacttc    180
   ttcaacaacc caggttctga cattcaaggt ttcattacct cttgtatggc tttgggttct    240
   ttcttcggtt ctattgtttc ttctttcatt tctgaaccat tcggtagaag agcttctttg    300
   ttgttgtgtt ctttcttctg gatggttggt gctgctgttc aatcttcttc tcaaaacaga    360
   gctcaattga tgattggtag aattattgct ggtttcggtg ttggtttcgg ttcttctgtt    420
   gctccagttt acggttctga attggctcca agaaagatta gaggtttcgt tggtggtatt    480
   ttccaattct gtgttacctt gggtattttg attatgttct acatttgtta cggtttgcac    540
   ttcattaacg gtgttggttc tttcagaatt gcttggggtt tgcaaattgt tccaggtttg    600
   gttttgttcg ttggttgttt cttcattcca gaatctccaa gatggttggc taagcacggt    660
   tactgggacg aagctgaatt cattgttgct caaattcaag ctaagggtaa cagagaagac    720
   ccagacgttt tgattgaaat ttctgaaatt aaggaccaaa ttttgattga agaaaacttg    780
   aagtctttcg gttacgttga cttgttcacc aagaagtaca ttagaagaac cttgaccgct    840
   attttcgctc aaatttggca acaattgacc ggtatgaacg ttatgatgta ctacattgtt    900
   tacattttca acatggctgg ttactctaac aacgctaact tggttgcttc ttctattcaa    960
   tacgttttga acaccgctgc taccgttcca gctttgttct tgatggacta cattggtaga   1020
   agaagattgt tgattggtgg tgctattatg atgatgattt tccaattcgg tgttgctggt   1080
   attttgggta agtactctgt tccagttcca ggtggtttgc caggtaaccc aaccgttacc   1140
   attcaaattc cagaagacaa caagtctgct gctagaggtg ttattgcttg ttgttacttg   1200
   ttcgttgttt ctttcgcttt gtcttggggt gttggtattt gggtttactg ttctgaagtt   1260
   tggggtgact ctgcttctag acaaagaggt gctgctgttt ctaccgctgc taactggatt   1320
   ttgaacttcg ctattgctat gtacacccca tcttctttca agaacattac ctggaagacc   1380
   tacattattt acgctgtttt ctgtttggtt atggctattc acgtttactt cggtttccca   1440
   gaaaccaagg gtaagagatt ggaagaagtt ggtcaaatgt gggacgaaaa cgttccagct   1500
   tggagatctt cttcttggca accaaccgtt ccattgttgt ctgacgctga cttggctcac   1560
   aagatggacg tttctcacaa ggaagaacaa tctccagacg ctgaatcttc ttctgaagaa   1620
   aagccataa                                                           1629
<210> 4
   <211> 2430
   <212> DNA
   <213> Pichia stipitis
<400> 4
   acaattttgt ggaataatcc cccaccttga taatccttct taggaacaat aaccggcgag      60
   atgtgcgggg cacgtcagga aacttaacct tgtcgcccta aattgccgtg aaggagagcc     120
   atacgatgga actccaaact tttatagtac atatagagtg gagtttgttt ggggaagtgg     180
   gaccatgggt caggaatttt gccgaaaaag ggcggttttg ttcactccta gtgcataatg     240
   cctgtgtata gacgatcaac ttgtattaat gtctgcatac cccaaatatt cacaattgac     300
   ccgcatcaaa aaataattct atctgacatc acgtatgcaa atacagtgag gaaatccatt     360
   cttgctgcca actcttcgcc tttttactca tccttgaaga aggaatcttt gcaatataaa     420
   agtaagatga aattcctctt attttacaat gttttgtcaa gtctttaaga aatttactct     480
   atcgtattta atattatatc atgagctacg aagataaact cgttcaacct gccttgaagt     540
   tcaggacctt cttggacaga cttccaaaca tttacaatgt gtacattatt gcatctattt     600
   cctgtatttc aggtatgatg ttcggttttg atatttcatc tatgtctgct tttataggtg     660
   aagatgacta caagaacttt ttcaataatc caggctcaga catccaaggt tttatcactt     720
   cctgtatggc tttaggttct ttcttcggtt ccatagtctc ttccttcatt tccgaaccat     780
   ttggtagaag agcatccttg ttgttgtgtt cattcttctg gatggtcggt gctgctgtac     840
   aatcatcttc tcaaaacaga gcccaattga tgatcggacg tatcatcgct ggtttcggtg    900
   ttggttttgg ttcttctgtt gctccagttt acggttccga attggctcca agaaagatta    960
   gaggttttgt tggtggtatt ttccaattct gtgttacctt gggtatcttg attatgttct   1020
   acatttgtta cggtttgcat ttcattaacg gtgttggctc tttcagaatt gcttggggtt   1080
   tacaaattgt cccaggtttg gttttatttg tcggttgttt ctttattcca gaatctccaa   1140
   gatggttagc caaacatggt tactgggatg aagcagaatt catcgttgcc caaattcaag   1200
   ctaagggtaa tagagaagac ccagacgtgt tgattgaaat ctccgaaatc aaggaccaaa   1260
   ttttgattga agaaaacctc aagagtttcg gttacgtcga cttattcacc aagaagtata   1320
   tcagaagaac tttaactgcc atatttgctc aaatctggca acaattgacc ggtatgaatg   1380
   ttatgatgta ctatattgtc tacattttca acatggccgg ttactctaac aacgcaaact   1440
   tggttgcctc ttccatccaa tacgtcttga acactgctgc aactgttcca gctttgtttt   1500
   taatggatta cattggcaga agaagattgt tgattggagg tgccatcatg atgatgattt   1560
   tccaatttgg tgttgctggt atcttaggta aatactccgt ccccgttcca ggcggtcttc   1620
   caggtaaccc aactgttacc atccaaatcc cagaagacaa caagtcagct gctagaggtg   1680
   ttattgcttg ttgttactta ttcgttgtat cattcgctct gagttggggt gtcggtatct   1740
   gggtctactg ttcagaagtt tggggtgact ctgcttccag acagagaggt gctgctgttt   1800
   caactgctgc caactggatt cttaactttg ctattgccat gtacactcca tcttccttca   1860
   agaatatcac ctggaagact tacatcatct acgccgtctt ctgtcttgtt atggcaatcc   1920
   atgtctactt tggattccca gaaaccaagg gcaagcgttt ggaagaagtc ggacaaatgt   1980
   gggacgaaaa tgttcccgca tggagatctt ccagctggca accaactgtt ccattgttgt   2040
   cagatgccga cttggcacac aagatggatg tttcccacaa ggaagagcaa tctccagatg   2100
   ccgagtcaag ttctgaggaa aagccttaaa ctaaattgat atgaataaac ctgttgcaac   2160
   agttgtgtga agtcaattgt tcacgtctta caataatgtc tttatgaaat gctttaaaca   2220
   atgtgctata ttaatttatc tgtttactat cttctgtagt acttcatata catccattat   2280
   cgaagatact cttcgtagac caatacccta atctcgcctg tacttcactg attgctgctc   2340
   tgctttaggt cccttcgaca cttacttttt gttctcgaat atatgacttg ttcatcgccc   2400
   taccaccaac tgaatcattg gtccgctatt                                    2430

## Beschreibung

### 1. Zusammenfassung

Die vorliegende Erfindung bezieht sich auf ein Polypeptid, das eine neue spezifische Arabinose-Transporterfunktion aufweist, sowie auf dafür kodierende Nukleinsäuren. Die Erfindung bezieht sich des weiteren auf Wirtszellen, insbesondere modifizierte Hefe-Stämme, die die kodierenden Nukleinsäuren enthalten und das Polypeptid exprimieren und funktionell in die Plasmamembran integrieren und somit L-Arabinose aufnehmen können. Bei der Verwendung von modifizierten Wirtszellen, die weitere Proteine des Arabinose-Stoffwechselweges exprimieren, kann Arabinose durch diese Zellen fermentiert werden, insbesondere zu Ethanol. Die vorliegende Erfindung ist somit unter anderem bedeutsam im Zusammenhang mit der Produktion von Biochemikalien aus Biomasse, wie z.B. Bioethanol.

### 2. Hintergrund der Erfindung

Die Bier-, Wein- und Bäckerhefe *Saccharomyces cerevisiae* wird aufgrund ihrer Eigenschaft, Zucker zu Ethanol und Kohlendioxid zu fermentieren, schon seit Jahrhunderten für die Brot-, Wein- und Bierherstellung genutzt. In der Biotechnologie findet *S*. *cerevisiae* neben der Produktion von heterologen Proteinen vor allem in der Ethanolproduktion für industrielle Zwekke Verwendung. Ethanol wird in zahlreichen Industriezweigen als Ausgangssubstrat für Synthesen benutzt. Durch die immer knapper werdenden Ölvorkommen, die steigenden Ölpreise und den stetig steigenden weltweiten Benzinbedarf gewinnt Ethanol als Kraftstoffalternative mehr und mehr an Bedeutung.

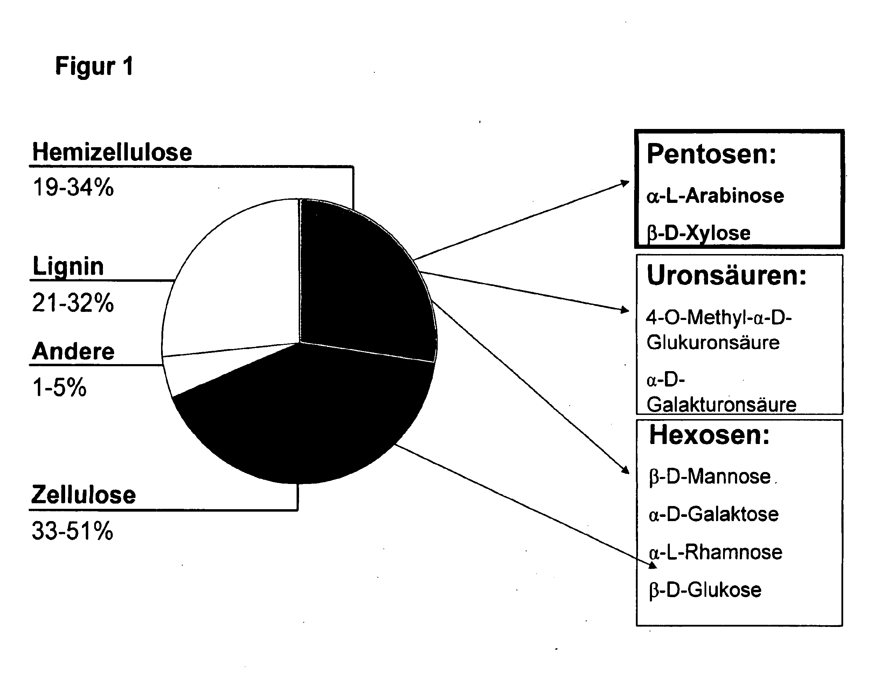

Um eine kostengünstige und effiziente Bioethanol-Produktion zu ermöglichen bietet sich die Verwendung von lignozellulosehaltiger Biomasse, wie z.B. Stroh, Abfälle aus der Holz- und Landwirtschaft und der organische Anteil aus dem alltäglichen Hausmüll, als Ausgangssubstrat an. Diese ist zum einen sehr günstig und zum anderen in großer Menge vorhanden. Die drei größten Bestandteile der Lignozellulose sind Lignin, Zellulose und Hemizellulose. Hemizellulose, nach Zellulose das am zweithäufigsten vorkommende Polymer, ist ein stark verzweigtes Heteropolymer. Es besteht aus Pentosen (L-Arabinose, D-Xylose), Uronsäuren (4-O-Methyl-D-Glucuronsäure, D-Galacturonsäure) und Hexosen (D-Mannose, D-Galactose, L-Rhamnose, D-Glucose) (siehe Figur 1). Hemizellulose lässt sich zwar leichter als Zellulose hydrolysieren, besitzt aber die Pentosen L-Arabinose und D-Xylose, die von der Hefe *S*. *cerevisiae* normalerweise nicht umgesetzt werden können.

Um Pentosen für Fermentationen nutzen zu können, müssen diese zuerst über die Plasmamembran in die Zelle hinein gelangen. Obwohl *S*. *cerevisiae* nicht in der Lage ist, D-Xylose zu metabolisieren, kann es diese in die Zelle aufnehmen. *S*. *cerevisiae* besitzt aber keine spezifischen Transporter. Der Transport findet mit Hilfe der zahlreichen Hexosetransporter statt. Die Affinität der Transporter zu D-Xylose ist aber deutlich niedriger als die zu D-Glucose (Kotter und Ciriacy, 1993). In Hefen, die D-Xylose metabolisieren können, wie z.B. *P. stipitis, C. shehatae* oder *P. tannophilus* (Du Preez et al., 1986), gibt es sowohl unspezifische niederaffine Transporter, die D-Glucose transportieren, als auch spezifische hochaffine Protonen-Symporter nur für D-Xylose (Hahn-Hagerdal *et al.,* 2001).

In früheren Versuchen konnten einige Hefen, wie zum Beispiel *Candida tropicalis, Pachysolen tannophilus, Pichia stipitis, Candida shehatae* gefunden werden, die von Natur aus L-Arabinose fermentieren oder zumindest assimilieren können. Diesen fehlt jedoch die Fähigkeit, L-Arabinose zu Ethanol zu fermentieren ganz oder sie besitzen nur eine sehr geringe Ethanolausbeute (Dien et al., 1996). Man weiß zudem über die Aufnahme von L-Arabinose noch sehr wenig. In der Hefe *C*. *shehatae* geht man von einem Protonen-Symport aus (Lucas und Uden, 1986). In *S*. *cerevisiae* ist von der Galactosepermease Gal2 bekannt, dass diese auch die in der Struktur zu D-Galactose ähnliche L-Arabinose transportiert (Kou *et al.,* 1970).

Alkoholische Fermentation von Pentosen in biotechnologisch veränderten Hefestämmen der *S. cerevisiae,* wobei unter anderem verschiedene Gene des Hefestammes *Pichia stipitis* zur genetischen Veränderung der *S*. *cerevisiae* herangezogen werden, wurde in den letzten Jahren vor allem in Zusammenhang mit der Fermentation von Xylose beschrieben. Dabei konzentrierte sich das Engineering vor allem auf die Einführung der Gene für die initiale Xylose-Assimilation aus *Pichia stipitis,* einer Xylose-fermentiemeden Hefe in *S*. *cerevisiae,* also in eine Hefe, die traditionell bei der Ethanol-Produktion aus Hexose eingesetzt wird (Jin et al. 2004).

Jeppson et al. (2006) beschreiben Xylose-Fermentation durch *S*. *cerevisiae* mittels Einführung eines Xylose-Stoffwechselweges, der entweder ähnlich zu dem in den Hefen *Pichia stipitis* und *Candida shehatae* ist, die natürlicherweise Xylose verwenden, oder ähnlich zum bakteriellen Stoffwechselweg ist.

Katahira et al. (2006) beschreiben Schwefelsäure-Hydrolysate von Lignozellulose-Biomasse, wie Holzspäne, als bedeutendes Material für die Produktion von Treibstoff-Bioethanol. In dieser Studie wurde ein rekombinanter Hefestamm konstruiert, der Xylose und Zellooligosaccharide fermentieren kann. Dazu wurden in diesen Hefestamm verschiedene Gene integriert, und zwar zur interzellulären Expression von Xylose-Reduktase und Xylitol-Dehydrogenase aus *Pichia stipitis* und Xylulokinase aus *S*. *cerevisiae* sowie zur Präsentation von Beta-Glucosidase aus *Aspergillus acleatus* auf der Zelloberfläche. Bei der Fermentation der Schwefelsäure-Hydrolysate von Holzspänen wurden Xylose und Zellooligosaccharide durch den rekombinanten Stamm nach 36 Stunden vollständig fermentiert.

Pitkanen et al. (2005) beschreiben die Gewinnung und Charakterisierung von Xylose-Chemostat-Isolaten eines *S*. *cerevisiae-Stammes,* der für Xylose-Reduktase und Xylitol-Dehydrogenase kodierende Gene aus *Pichia stipitis* and das Gen, das die endogene Xylulokinase kodiert, überexprimiert. Die Isolate wurden aus aeroben Chemostat-Kulturen auf Xylose als einzige oder hauptsächliche Kohlenstoffquelle gewonnen. Unter aeroben Bedingungen auf Minimalmedium mit 30 g/l Xylose war die Wachstumsrate der Chemostat-Isolate 3-fach höher als die des Originalstammes (0,15 h⁻¹ gegenüber 0,05 h⁻¹). Die Xylose-Aufnahmerate war fast 2-fach erhöht. Die Aktivitäten der Schlüsselenzyme des Pentosephosphat-Stoffwechselweges (Transketolase, Transaldolase) waren 2-fach erhöht, während die Konzentrationen ihrer Substrate (Pentose-5-phosphate, Sedoheptulose-7-phosphat) entsprechend erniedrigt waren.

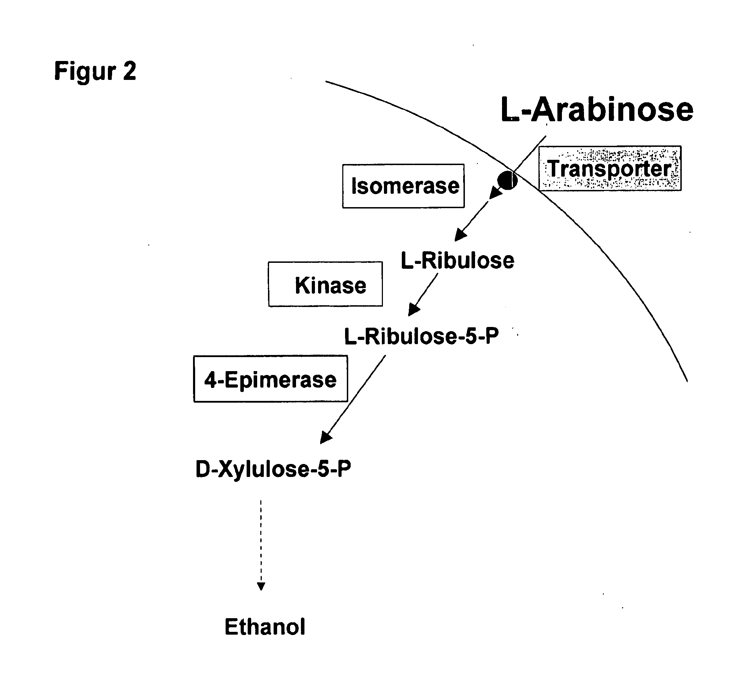

Becker und Boles (2003) beschreiben das Engineering und die Selektion eines Laborstammnes von *S*. *cerevisiae,* der in der Lage ist, L-Arabinose zum Wachstum zu verwenden und zu Ethanol zu fermentieren. Dies war möglich durch die Überexpression eines bakteriellen L-Arabinose-Stoffwechselweges, bestehend aus *Bacillus subtilis* AraA and *Escherichia coli* AraB and AraD and simultaner Überrexpression der L-Arabinose-transportierenden Hefe-Galaktose-Permease in dem Hefestamm. Molekulare Analyse des selektierten Stammes zeigte, dass die entscheidende Voraussetzung für eine Verwendung von L-Arabinose eine niedrigere Aktivität von L-Ribulokinase ist. Von diesem Hefestamm wird jedoch unter anderem ein sehr langsames Wachstum berichtet. (siehe Figur 2)

Es besteht also im Stand der Technik Bedarf für spezifische Pentose-Transporter, insbesondere L-Arabinose-Transporter, die es ermöglichen, Pentosen, insbesondere L-Arabinose spezifisch in Zellen, wie Hefezellen, aufzunehmen und somit eine Verwertung und Fermentation von Pentosen, insbesondere L-Arabinose zu begünstigen.

Aufgabe der vorliegenden Erfindung ist es daher, spezifische Pentose-Transporter, wie L-Arabinose-Transporter, zur Verfügung zu stellen.

Diese Aufgabe wird durch die Erfindung, wie in den Patentansprüchen beansprucht, gelöst.

Die Aufgabe wird erfindungsgemäß gelöst durch das zur Verfügung stellen von Polypeptiden, die eine *in vitro* und/oder *in vivo* Pentose-Transportfunktion aufweisen, und Varianten und Fragmenten davon.

Das erfindungsgemäße Polypeptid ist ausgewählt aus der Gruppe von
a. einem Polypeptid, das zu mindestens 70%, bevorzugt mindestens 80% identisch zu der Aminosäuresequenz nach SEQ ID NO:1 ist und eine *in vitro* und/oder *in vivo* Pentose-Transportfunktion aufweist, und
b. einem Polypeptid, das identisch zu der Aminosäuresequenz nach SEQ ID NO:1 1 ist und eine *in vitro* und/oder *in vivo* Pentose-Transportfunktion aufweist,
wobei die Pentose bevorzugt Arabinose, insbesondere L-Arabinose ist.

Bevorzugterweise umfasst das erfindungsgemäße Polypeptid ein Fragment von mindestens 200 oder 300 kontinuierlichen Aminosäuren nach SEQ ID NO:1. Dabei wird ein solches Fragment dadurch charakterisiert, dass es eine *in vitro* und/oder *in vivo* Pentose-Transportfunktion aufweist.

In einer bevorzugten Ausführungsform umfasst ein erfindungsgemäßes Polypeptid ein Fragment von 502 Aminosäuren, das den ersten 502 Aminosäuren nach SEQ ID NO: 1 entspricht. Ein solches Fragment ist dadurch charakterisiert, dass es eine *in vitro* und/oder *in vivo* Pentose-Transportfunktion aufweist.

Bevorzugterweise ist das erfindungsgemäße Polypeptid ein Polypeptid das zu mindestens 90%, bevorzugt 95%, weiter bevorzugt zu 99% identisch zu der Aminosäuresequenz nach SEQ ID NO:1 ist und eine *in vitro* und/oder *in vivo* Pentose-Transportfunktion aufweist.

Varianten der erfindungsgemäße Polypeptide können auch solche sein, die konservative Aminosäure-Austausche aufweisen oder kleinere Deletionen und/oder Insertionen, solange diese Veränderungen die *in vitro* und/oder *in vivo* Pentose-Transportfunktion nicht wesentlich beeinflussen.

Erfindungsgemäße Polypeptide können weiterhin heterologe Aminosäuresequenzen umfassen. Geeignete heterologe Aminosäuresequenzen kann der Fachmann je nach Anwendung oder Verwendung selbst auswählen.

Bevorzugterweise ist die Pentose Arabinose, insbesondere L-Arabinose, so dass ein erfindungsgemäßes Polypeptid bevorzugt eine *in vitro* und/oder *in vivo* Arabinose-Transportfunktion, insbesondere eine L-Arabinose-Transportfunktion aufweist.

Das erfindungsgemäße Polypeptid stammt bevorzugt aus einer Hefe, bevorzugt aus *Pichia,* insbesondere *Pichia stipitis,* ab.

Die Aufgabe wird weiterhin erfindungsgemäß durch das zur Verfügung stellen von isolierten Nukleinsäuremolekülen, die für ein erfindungsgemäßes Polypeptid kodieren, gelöst.

Bevorzugterweise ist ein erfindungsgemäßes Nukleinsäuremolekül zu mindestens 90%, bevorzugt 95% und weiter bevorzugt 99% identisch zu der Nukleinsäuresequenz nach SEQ ID NO:2 oder 3.

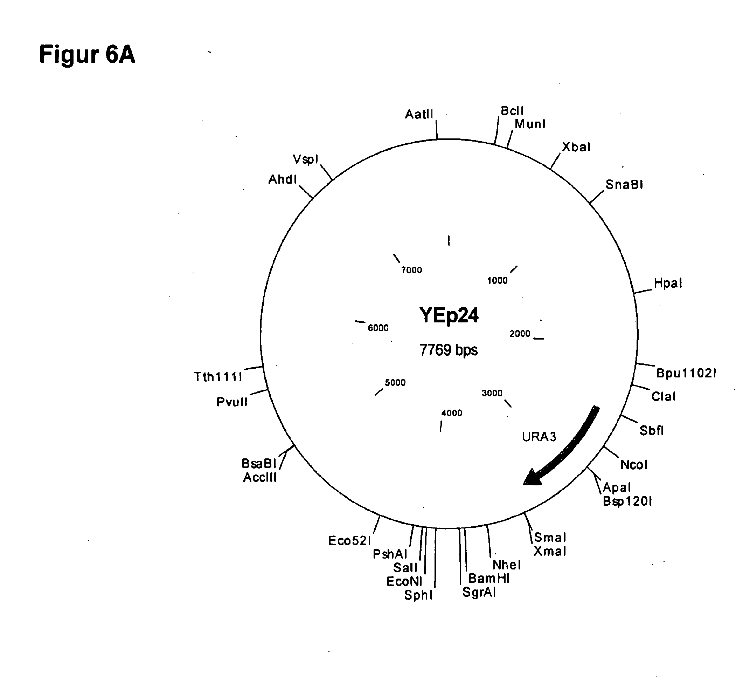

Ein erfindungsgemäßes Nukleinsäuremolekül umfasst weiter Vektor-Nukleinsäuresequenzen, bevorzugt Expressionsvektor-Sequenzen. Vektor-Nukleinsäuresequenzen sind bevorzugt ausgewählt aus Sequenzen, die von den Vektoren der Gruppe bestehend aus YEp24, p426HXT7-6HIS, p426Met25, pYES260, pYES263, pVTU260, pVTU263, pVTL260, pVTL263 umfasst werden. Für weitere Ausführungen siehe Figuren 6A-E und Beispiel 3.

Erfindungsgemäße Nukleinsäuremoleküle können weiterhin Nukleinsäuresequenzen, die für weitere heterologe Polypeptide kodieren, umfassen. Geeignete heterologe Nukleinsäuresequenzen, die für weitere heterologe Polypeptide kodieren, kann der Fachmann je nach Anwendung oder Verwendung selbst auswählen. Dazu zählen beispielsweise Antibiotika-Resistenzmarker-Sequenzen.

Erfindungsgemäße Nukleinsäuremoleküle umfassen bevorzugt dsDNA, ssDNA, PNA, CNA, RNA oder mRNA oder Kombinationen davon.

Die Aufgabe wird weiterhin erfindungsgemäß gelöst durch das zur Verfügung stellen von Wirtszellen, die mindestens ein erfindungsgemäßes Nukleinsäuremolekül enthalten. Erfindungsgemäße Wirtszellen exprimieren dieses mindestens eine erfindungsgemäße Nukleinsäuremolekül zudem bevorzugt.

Eine erfindungsgemäße Wirtszelle ist insbesondere eine Pilzzelle und bevorzugt eine Hefezelle, wie *Saccharomyces* species, z.B. *S*. *cerevisiae, Kluyveromyces* sp., z.B. *K. lactis, Hansenula* sp., z.B. *H. polymorpha, Pichia* sp. , z.B. *P. pastoris, Yarrowia* sp., z.B. *Y. lipolytica.*

Bevorzugterweise enthalten erfindungsgemäße Wirtszellen weiterhin Nukleinsäuremoleküle, die für Proteine des Arabinose-Stoffwechselweges kodieren, insbesondere für L-Ribulokinase, L-Ribulose-5-P 4-Epimerase, L-Arabinose-Isomerase.

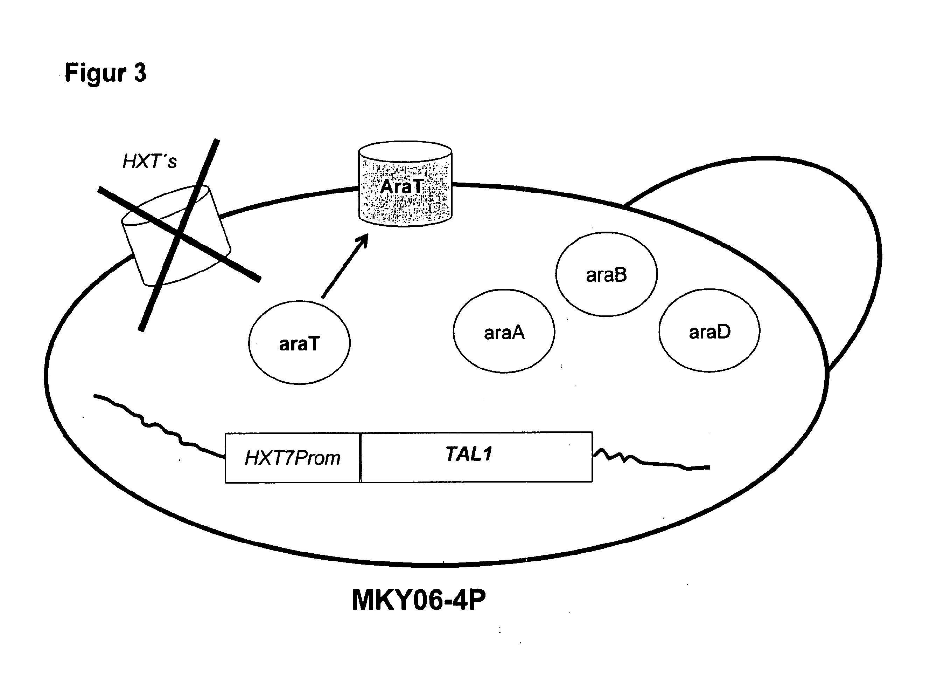

Dabei handelt es sich bevorzugt um Proteine des bakteriellen Arabinose-Stoffwechselweges, insbesondere *E*. *coli araB* L-Ribulokinase, *E.coli araD* L-Ribulose-5-P 4-Epimerase und *B*. *subtilis araA* L-Arabinose-Isomerase. Siehe auch Figuren 2 und 3.

Besonders bevorzugte Wirtszellen dieser Erfindung sind Zellen des Stammes MKY06-4P der bei der Deutschen Sammlung von Mikroorganismen und Zellkulturen am 23. August 2006 unter der Hinterlegungsnummer DSM 18544 hinterlegt wurde. Siehe auch Figur 3.

Eine bevorzugte Wirtszelle gemäß dieser Erfindung ist eine Hefezelle, die durch Einführung und Expression der Gene araA (L-Arabinose-Isomerase), araB (L-Ribulokinase) und araD (L-Ribulose-5-P-4-epimerase) modifiziert wurde und zudem ein TAL1 (Transaldolase) -Gen überexprimiert, wie beispielsweise von den Erfindern in der EP 1 499 708 B1 beschrieben, und zusätzlich dazu wenigstens ein erfindungsgemäßes Nukleinsäuremolekül enthält.

Die Aufgabe wird weiterhin erfindungsgemäß gelöst durch das zur Verfügung stellen von Antikörpern oder Antikörper-Fragmenten, umfassend einen immunologisch aktiven Teil, der selektiv an ein erfindungsgemäßes Polypeptid bindet. Verfahren zur Generation von Antikörpern oder Antikörper-Fragmenten sind im Stand der Technik bekannt.

Die Aufgabe wird weiterhin erfindungsgemäß gelöst durch Verfahren zur Herstellung eines erfindungsgemäßen Polypeptids. Ein solches Verfahren umfasst das Kultivieren einer erfindungsgemäßen Wirtszelle unter Bedingungen, unter denen ein erfindungsgemäßes Nukleinsäuremolekül exprimiert wird. Allgemeine Verfahren zur Generation von Polypeptiden mittels Zellkultur sind im Stand der Technik bekannt.

Die Aufgabe wird weiterhin erfindungsgemäß gelöst durch einen Kit, umfassend eine Verbindung, die selektiv an ein erfindungsgemäßes Polypeptid bindet, gegebenenfalls mit weiteren Hilfsstoffen und Gebrauchsanweisungen.

Die Verbindung ist bevorzugt eine Pentose, wie zum Beispiel Arabinose, und insbesondere L-Arabinose, oder ein Derivat einer solchen Pentose.

Die Aufgabe wird weiterhin erfindungsgemäß gelöst durch Verfahren zur Identifizierung einer Verbindung, die an ein erfindungsgemäßes Polypeptid bindet und/oder seine Aktivität moduliert. Ein solches Verfahren umfasst die folgenden Schritte:

In Kontakt bringen eines Polypeptids oder einer Zelle, die ein erfindungsgemäßes Polypeptid exprimiert, mit einer Testverbindung, und
Bestimmen, ob das Polypeptid an die Testverbindung bindet und, gegebenenfalls, Bestimmen, ob die Testverbindung die Aktivität des Polypeptids moduliert.

Die Verbindung ist bevorzugt eine Pentose, wie zum Beispiel Arabinose, und insbesondere L-Arabinose, oder ein Derivat einer solchen Pentose.

Die Aufgabe wird weiterhin erfindungsgemäß gelöst durch Verfahren zur Modulierung der Aktivität eines erfindungsgemäßen Polypeptids. Ein solches Verfahren umfasst in Kontakt bringen eines Polypeptids oder einer Zelle, die ein erfindungsgemäßes Polypeptid exprimiert, mit einer Verbindung, die an das Polypeptid bindet in einer ausreichenden Konzentration, um die Aktivität des Polypeptids zu modulieren.

Die.Verbindung ist bevorzugt eine Pentose, wie zum Beispiel Arabinose, und insbesondere L-Arabinose, oder ein Derivat einer solchen Pentose.

Die Aufgabe wird weiterhin erfindungsgemäß gelöst durch Verfahren zur Produktion von Bioethanol. Ein solches erfindungsgemäßes Verfahren umfasst die Expression eines erfindungsgemäßen Nukleinsäuremoleküls in einer erfindungsgemäßen Wirtszelle.

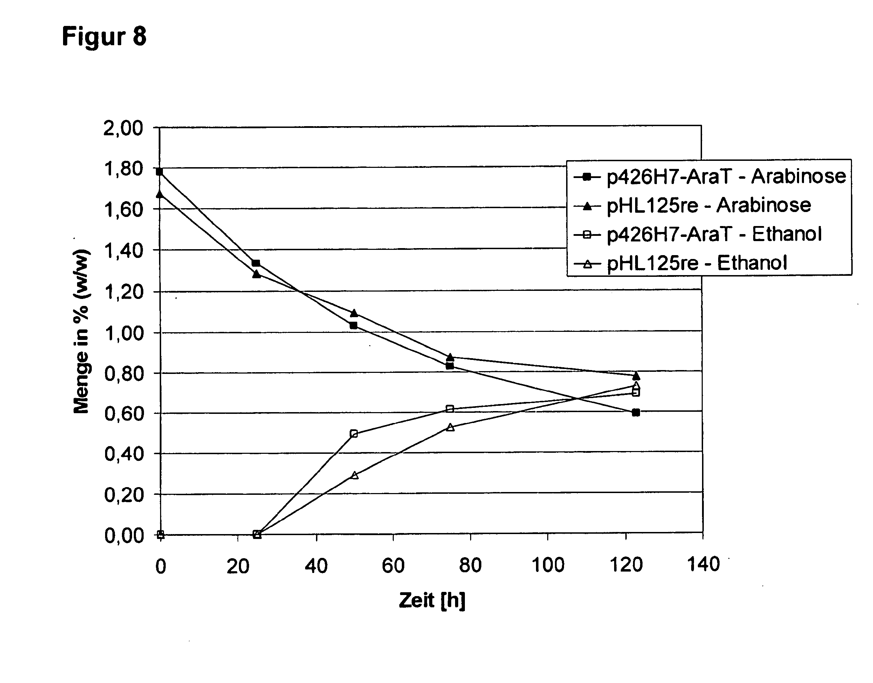

Die erfindungsgemäßen Polypeptide, Nukleinsäuremoleküle und Wirtszellen werden insbesondere bevorzugt zur Produktion von Bioethanol verwendet. Für bevorzugte Ausführungsformen wird auf Figur 8 und Beispiel 4 verwiesen.

Die erfindungsgemäßen Polypeptide, Nukleinsäuremoleküle und Wirtszellen werden weiter insbesondere bevorzugt zur rekombinanten Fermentation von Pentose-haltigem Biomaterial verwendet.

Spezifische Gene aus *Pichia stipitis,* die die Aufnahme der Pentose L-Arabinose in *S*. *cerevisiae* spezifisch erhöhen, wurden unter Verwendung einer Genbank isoliert und in den Hefestamm MKY06-3P integriert, der dann in der Lage ist, die L-Arabinose zu Ethanol zu vergären. Das Screening der relevanten Gene führte zu einem neuen spezifischen L-Arabinose-Transporter, dessen Nukleotid- und Proteinsequenz vorliegt (siehe SEQ ID NOs: 1-4). Dazu wird auch auf die Beispiele und Figuren verwiesen.

Aufgrund der Spezifität dieses neuen Transporters kann nach Expression in bestehenden Ethanol-produzierenden Systemen die Aufnahmerate für L-Arabinose verbessert werden, da zum einen bei hohen L-Arabinose-Konzentrationen die Konkurrenzsituation gegenüber Glukose verbessert wird und zum anderen bei niedrigen L-Arabinose-Konzentrationen aufgrund einer hohen Affinität der Transport von L-Arabinose effizienter wird.

### Aufnahme von L-Arabinose

Damit die Pentose L-Arabinose von *S*. *cerevisiae* metabolisiert werden kann, muss diese zuerst von der Zelle aufgenommen werden. Bezüglich dieser Aufnahme ist erst wenig bekannt. Man kennt bisher in Eukaryonten noch keine Gene, die für spezifische L-Arabinose-Transporter kodieren. Alle für die Pentose D-Xylose getesteten Hexosetransporter besitzen eine viel höhere Affinität zu D-Glucose als zu D-Xylose. Für L-Arabinose wird ähnliches vermutet. Von allen bisher konstruierten Stämmen, die Pentosen (D-Xylose oder L-Arabinose) verwerten können, wird ein relativ langsames Wachstum berichtet. Als Grund hierfür wird vor allem die langsame und schlechte Aufnahme der Pentosen genannt (Becker und Boles, 2003; Richard *et al.,* 2002). Bei Fermentationen in einem Zuckergemisch, bestehend aus D-Glucose und D-Xylose oder D-Glucose und L-Arabinose, werden die Zucker nicht simultan umgesetzt. Durch die hohe Affinität der Transporter zu D-Glucose wird diese zuerst metabolisiert. Es tritt ein so genannter Diauxie-Shift ein. Erst nachdem die D-Glucose aufgebraucht ist, wird die Pentose in einer zweiten deutlich langsameren Wachstumsphase umgesetzt (Kuyper *et al.,* 2005a; Kuyper *et al.,* 2005b). Als eine Erklärung wird das Fehlen von spezifischen Transportern für Pentosen angeführt.

### Neuer spezifischer L-Arabinose Transporter aus P. stipitis

Für industrielle Anwendungen wäre es ideal, wenn der verwendete Mikroorganismus alle im Medium vorhandenen Zucker möglichst gleichzeitig umsetzen könnte (Zaldivar *et al.,* 2001). Um dies zu erreichen, wären spezifische Transporter für jeden Zuckertyp von großem Nutzen. Besonders für L-Arabinose kannte man bisher noch keinen.

In dieser Erfindung ist es mit einem Testsystem (siehe Beispiele) gelungen, ein spezifisches L-Arabinose-Transportergen aus dem Genom von *P. stipitis* zu finden. Auf den Plasmiden pAraT1 und pAraT7 sind Genomfragmente aus *P. stipitis* lokalisiert, welche für ein spezifisches Wachstum auf L-Arabinose aber nicht auf D-Glucose, D-Mannose oder D-Galactose Medium verantwortlich sind. Das beobachtete geringe Wachstum auf D-Galactose wurde nicht von den Plasmiden pAraT1 oder pAraT7 verursacht. Hierbei handelte es sich nur um das schwache Wachstum des EBY.VW4000, dem Ausgangstamm des MKY06, welches bereits von Wieczorke *et al.* (1999) berichtet wurde. Die Möglichkeit, dass das erhaltene Wachstum durch eine genomische Mutation im MKY06 bewirkt wurde, konnte ausgeschlossen werden. Nach einer Selektion auf den Verlust des Plasmids der *P. stipitis* Genbank durch zweimaligen Ausstrich auf FOA-Medium wurde bei erneutem Ausstreichen auf L-Arabinose Medium kein Wachstum mehr festgestellt. Somit rührte das Wachstum von den Plasmiden der *P. stipitis* Genbank her (siehe Beispiele). Es konnte gezeigt werden, dass die gefundenen Plasmide einen Transporter codieren.

Bei einer BLAST-Recherche mit dem kürzlich veröffentlichten Genom von *Pichia stipitis* (siehe http://genome.jgi-psf.org/euk_home.html) zeigte sich eine Übereinstimmung von 100 % mit HGT2. HGT2 wurde aufgrund seiner hohen Homologie zum hochaffinen Glucose-Transporter HGT1 von *Candida albicans* als putativer hochaffiner Glucose-Transporter annotiert. Wenn man die Sequenz hinsichtlich der möglichen Transmembran-Domänen untersucht, erhält man 12 Transmembran-Domänen, was für Transporter typisch ist. Es ist daher überraschend, dass es sich um einen Pentose-Transporter (Arabinose-Transporter) und nicht um einen Hexose-Transporter handelt.

Des weiteren waren eine Vielzahl von experimentellen Hürden und Schwierigkeiten beim Auffinden und Bereitstellen des erfindungsgemäßen Transporters zu überwinden, die sich in größerem Detail auch aus den Beispielen und Figuren ergeben.
- Im Ausgangsstamm EBY.VW4000 mussten insgesamt 21 Monosaccharid-Transporter Gene deletiert werden.
- Weiterhin musste in diesem Stamm *TAL1* genomisch überexprimiert werden.
- Die Etablierung der optimalen Wachstumsbedingungen für die Durchführung des Screens gestaltete sich sehr schwierig und zeitaufwendig.
- Bei dem erfindungsgemäßen Transporter handelt es sich um den ersten beschriebenen spezifischen Arabinose-Transporter von Eukaryonten
- Es handelt sich um einen heterolog exprimierten und zugleich funktionell in der Plasmamembran von *S*. *cerevisiae* eingebauten Transporter, was nicht unbedingt zu erwarten ist.

Über die Schwierigkeiten bezüglich heterolog exprimierter Transporter gibt es einige Berichte, siehe dazu Chapter 2 im "Buch Transmembrane Transporters" (Boles, 2002) und den Artikel von Wieczorke et al., 2003.

Weitere Biomasse mit signifikanten Mengen an Arabinose (Quelle der Daten: U.S. Department of Energy http://www.eere.energy.gov/biomass/progs/searchl.cgi):

| Art der Biomasse | L-Arabinose [%] |
|---|---|
| Switchgras | 3,66 |
| Grosses Bartgras | 3,55 |
| Hohes Schwingelgras | 3,19 |
| Robinie | 3 |
| Corn Stover | 2,69 |
| Weizen Stroh | 2,35 |
| Zuckerrohr Bagasse | 2,06 |
| Chinesischer Buschklee | 1,75 |
| Futterhirse | 1,65 |

Auch für deren Verwertung ist der erfindungsgemäße Arabinose-Transporter von großer Bedeutung.

Nutzungsmöglichkeiten eines in der Hefe *S*. *cerevisiae* funktionellen und zugleich spezifischen Arabinose-Transporters sind zum einen die Produktion von Bioethanol und die Herstellung von hochwertigen Vorläufer-Produkten für weitere chemische Synthesen.

Folgende Auflistung stammt aus der Studie "Top Value Added Chemicals From Biomass" (siehe wwwl.eere.energy.gov/biomass/pdfs/35523.pdf). Hierbei wurden 30 Chemikalien als besonders wertvoll eingestuft, welche aus Biomasse hergestellt werden können.

| Anzahl der C-Atome | Top 30 Kandidaten |
|---|---|
| 1 | Wasserstoff, Kohlenmonoxid |
| 2 | |
| 3 | Glycerol, 3-Hydroxypropionsäure, Milchsäure, Malonsäure, Propionsäure, Serin |
| 4 | Acetoin, Asparaginsäure, Fumarsäure, 3-Hydroxybutyrolacton, Apfelsäure, Bernsteinsäure, Threonin |
| 5 | Arabitol, Furfural, Glutaminsäure, Itaconsäure, Levulinsäure, Prolin, Xylitol, Xylonsäure |
| 6 | Aconitsäure, Citrat, 2,5-Furandicarboxylsäure, Glucaricsäure, Lysin, Levoglucosan, Sorbitol |

Sobald diese Chemikalien aus Lignozellulose durch Biokonversion (z.B. Fermentationen mit Hefen) hergestellt werden, ist es wichtig, einen spezifischen Transporter für die Hemizellulose Arabinose zu besitzen.

Die vorliegende Erfindung wird in den folgenden Figuren, Sequenzen und Beispielen weiter verdeutlicht, ohne jedoch darauf beschränkt zu sein, Die zitierten Referenzen sind hiermit durch Bezugnahme vollständig aufgenommen. In den Sequenzen und Figuren zeigt:
SEQ ID NO: 1 die Proteinsequenz des AraT-ORF's,
SEQ ID NO: 2 die Sequenz des offenen Leserahmens (ORF) von AraT,
SEQ ID NO: 3 die Sequenz des offenen Leserahmens (ORF) von AraT in einer Codonoptimierten Form, und
SEQ ID NO: 4 die Sequenz des offenen Leserahmens (ORF) von AraT mit 500 Promotor, ORF und 300 Terminator.

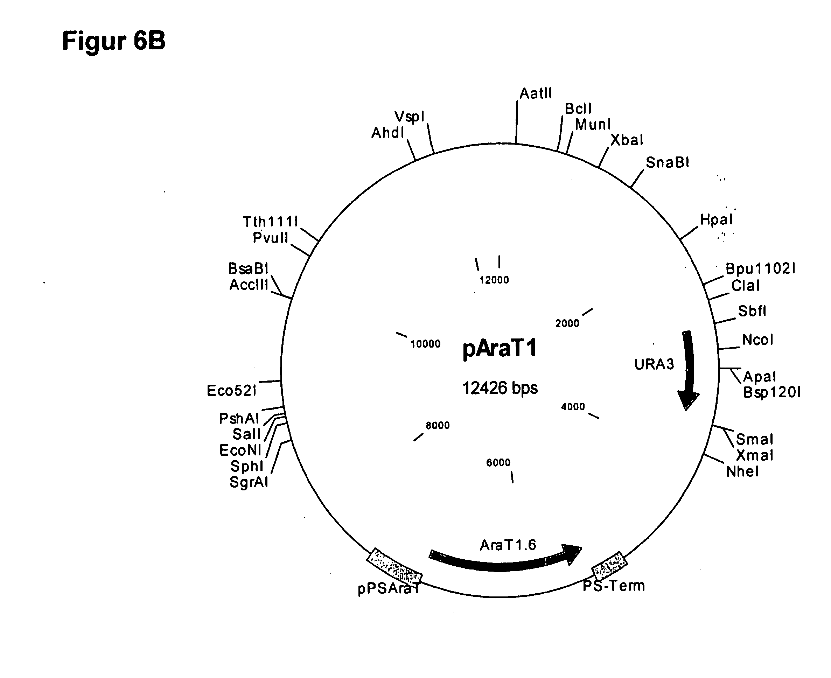

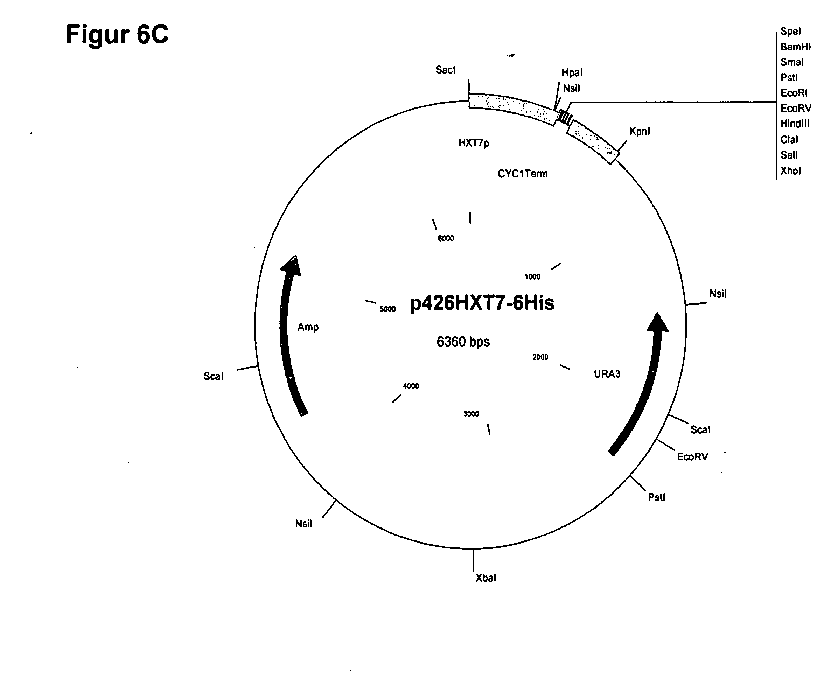

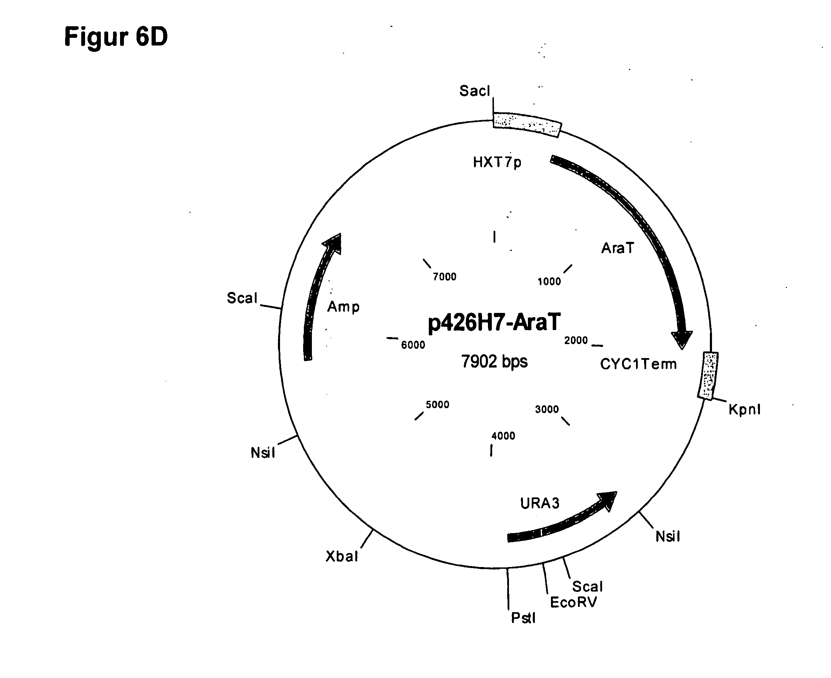

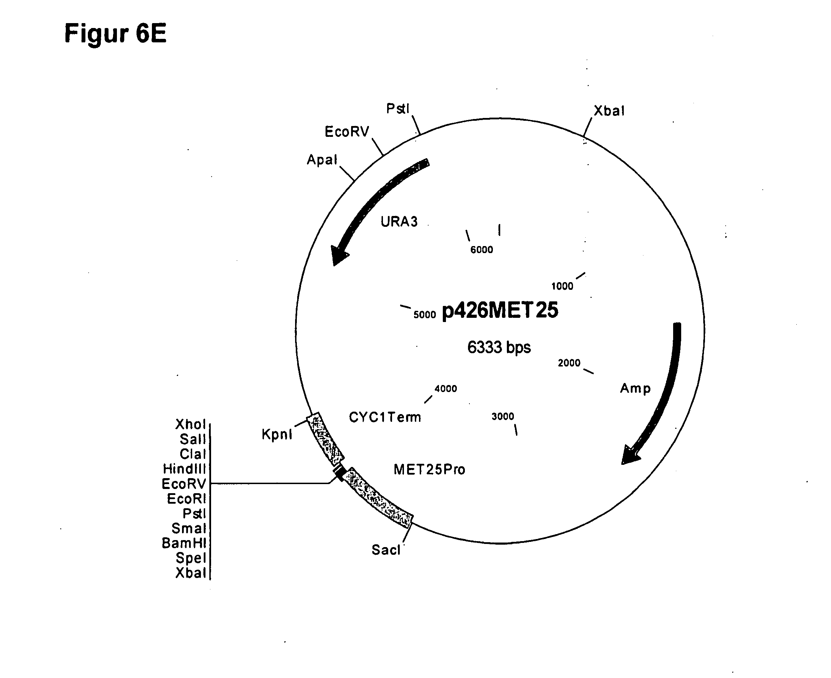

**Figur 1****.** *Zusammensetzung der Biomasse.*
   Die am zweithäufigsten vorkommende Hemizellulose ist ein aus Pentosen, Uronsäuren und Hexosen bestehender stark verzweigter Polymer. Die Hemizellulose besteht zu einem großen Anteil aus den Pentosen Xylose und Arabinose.
**Figur 2****.** *Schema für die Verwendung von L-Arabinose in rekombinanter S. cerevisiae durch Integration eines bakteriellen L-Arabinose-Stoffwechselweges.*
**Figur 3****.** *Konstruktion des erfindungsgemäßen Hefestammes MKY06-4P.*
   Ausgangsstamm für die Konstruktion des MKY06-4P war der Hefestamm EBY.VW4000, in dem alle Hexosetransportergene (*HXT's*) deletiert wurden. In diesem Stamm wurde die endogene Transaldolase *TAL1* durch den Austausch des nativen Promotors gegen den verkürzten *HXT7*-Promotor (*HXT7-Prom*) überexprimiert. Dies führte zu dem Stamm MKY06. In diesem Stamm wurden die Plasmide p423H7araABs^{re} (araA), p424H7araB^{re} (araB) und p425H7araD^{re} (araD) für den Arabinose-Stoffwechsel hineintransformiert (=MKY06-3P). Zusätzlich wurde noch das Plasmid p426H7-araT (araT), welches den erfindungsgemäßen Arabinose-Transporter aus *Pichia stipitis* kodiert hineintransformiert und so der Stamm MKY06-4P erhalten. Der Transporter wird exprimiert und funktionell in die Plasmamembran eingebaut (AraT).

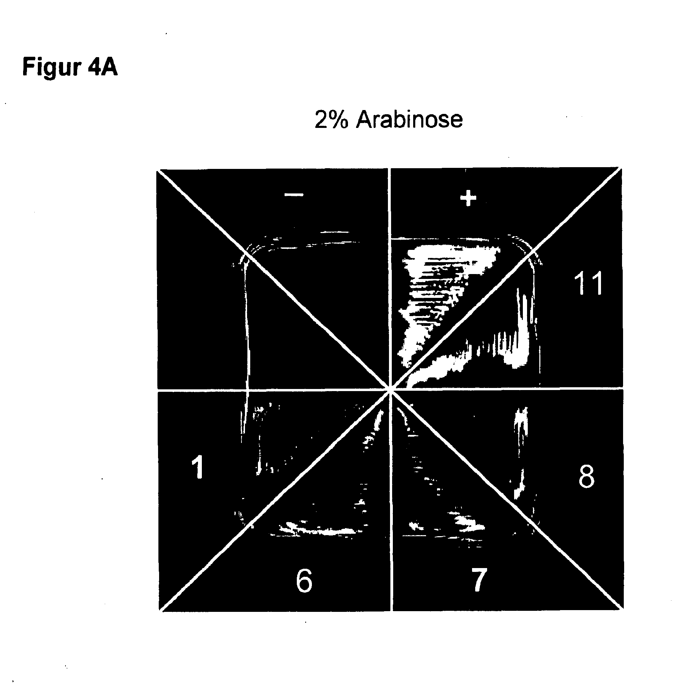

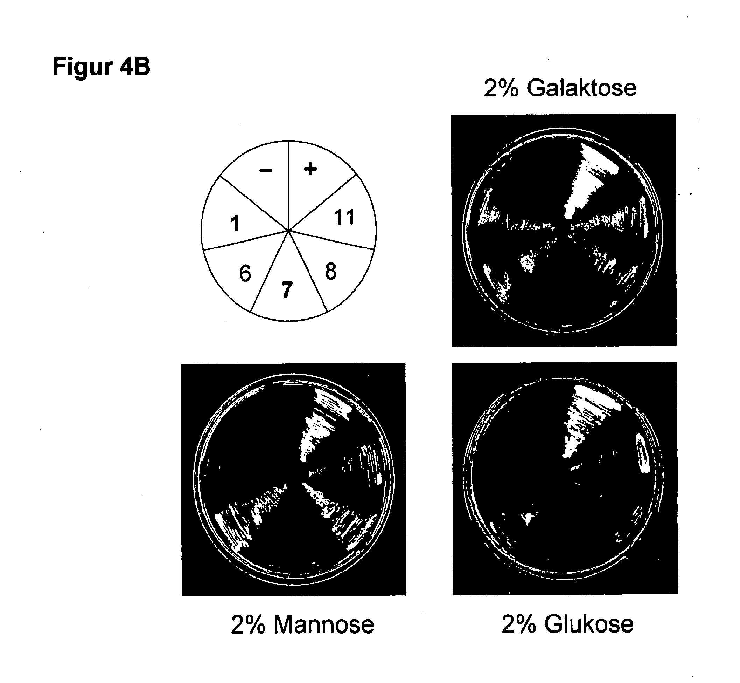

**Figur 4****.** *Ausstrich des MKY06 mit den Plasmiden für den L-Arabinose Stoffwechsel und den gefundenen L-Arabinose-Transportern auf unterschiedlichen Kohlenstoffquellen.*
   Es handelte sich bei jedem Ausstrich um den MKY06-3P und zusätzlich einem Plasmid aus der Genbank YEpTW. Als Negativkontrolle (-) wurde anstatt eines Plasmids der Genbank das p426HXT7-6HIS und als Positivkontrolle (+) das pHL125^{re} hineintransformiert.^{.} 1: pAraT1, 6: pAraT6, 7: pAraT7, 8: pAraT8, 11: pAraT11, -: Negativkontrolle, +: Positivkontrolle.
   **A:** Medium 2% L-Arabinose
   **B:** Medium jeweils 2% D-Galactose, D-Glucose oder D-Mannose
   Alle SC-Mediumsplatten wurden bei 30°C inkubiert. Die L-Arabinose-Platte (A) zeigt das Wachstum nach 9 und alle anderen Platten (B) nach 2 Tagen. Die Kolonien 1 und 7 wuchsen auf L-Arabinose aber nicht auf D-Glucose, D-Mannose und nur schwach auf D-Galactose.

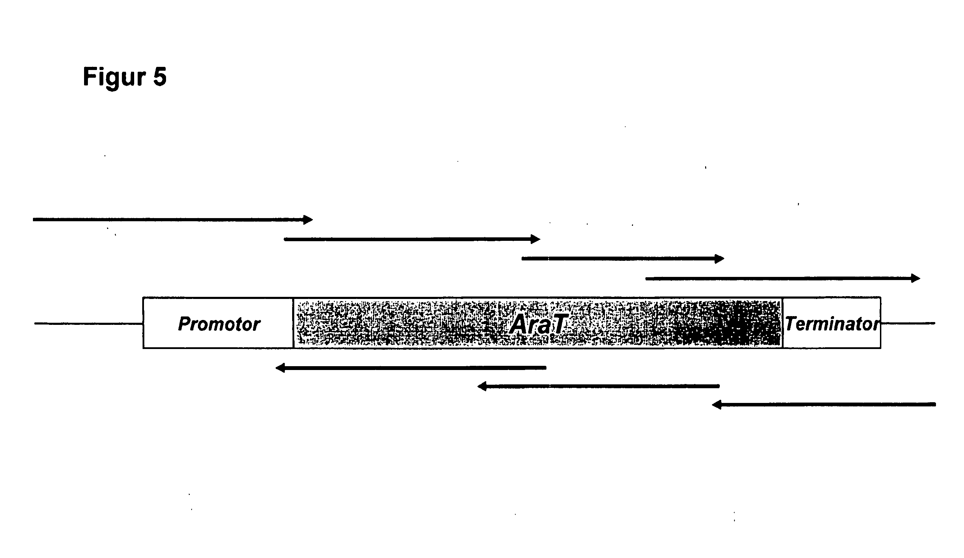

**Figur 5****.** *Sequenzierung des Arabinose-Transporters.*
   Der komplette offene Leserahmen des erfindungsgemäßen Transporters wurde doppelsträngig mit überlappenden Bereichen sequenziert. Der Promotor und Terminator-Bereich wurde einzelsträngig sequenziert. Die Pfeile zeigen die Bereiche von einzelnen Sequenzierungen an
**Figur 6****.** *Verwendete Vektoren und deren Aufbau.*
   Ausgangsplasmid für die Herstellung der *P. stipitis* Genbank war das Plasmid YEp24 (A). Das Plasmid pAraT1 (B) als auch das Plasmid pAraT7 basieren somit auf dem YEp24 und unterscheiden sich nur in der Größe des Inserts. Der offene Leserahmen (ORF) des erfindungsgemäßen Arabinose-Transporters wurde von dem pAraT1 amplifiziert und hinter den verkürzten starken *HXT7*-Promotor des Plasmids p426HXT7-6HIS (C) kloniert. Hierbei entstand das Plasmid p426H7-AraT (D), welches einen Uracil-Marker besitzt. Ein anderes mögliches Expressionsplasmid ist p426Met25 (E).

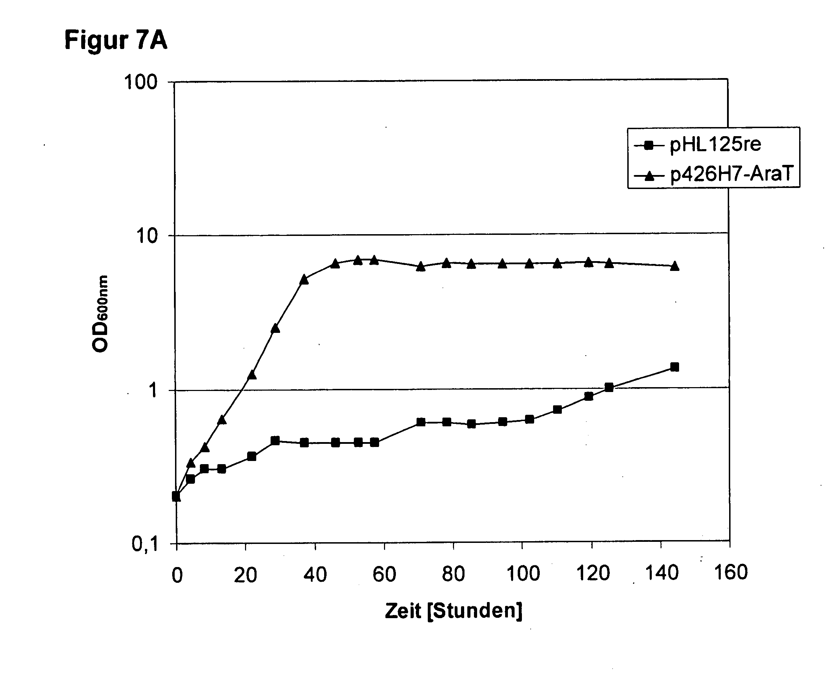

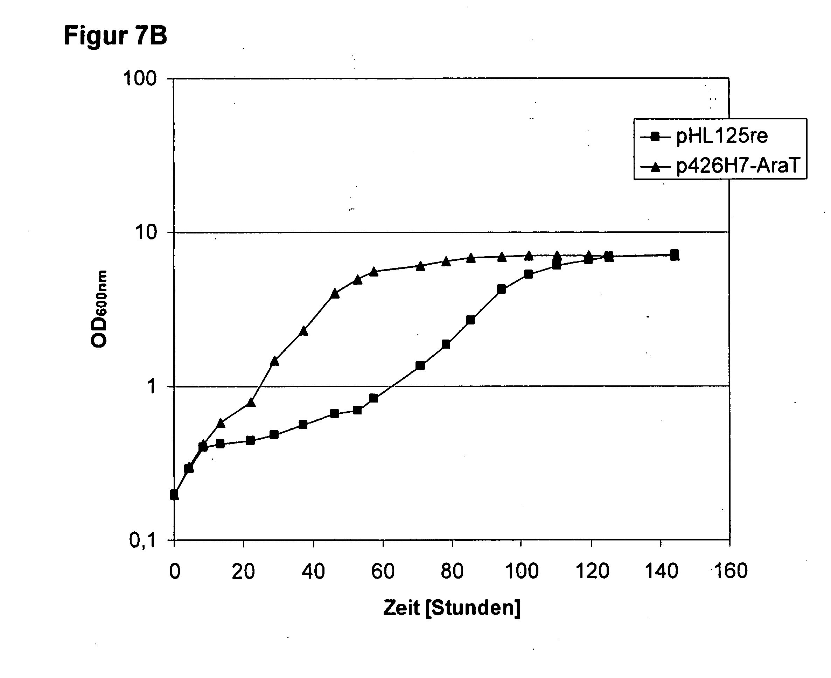

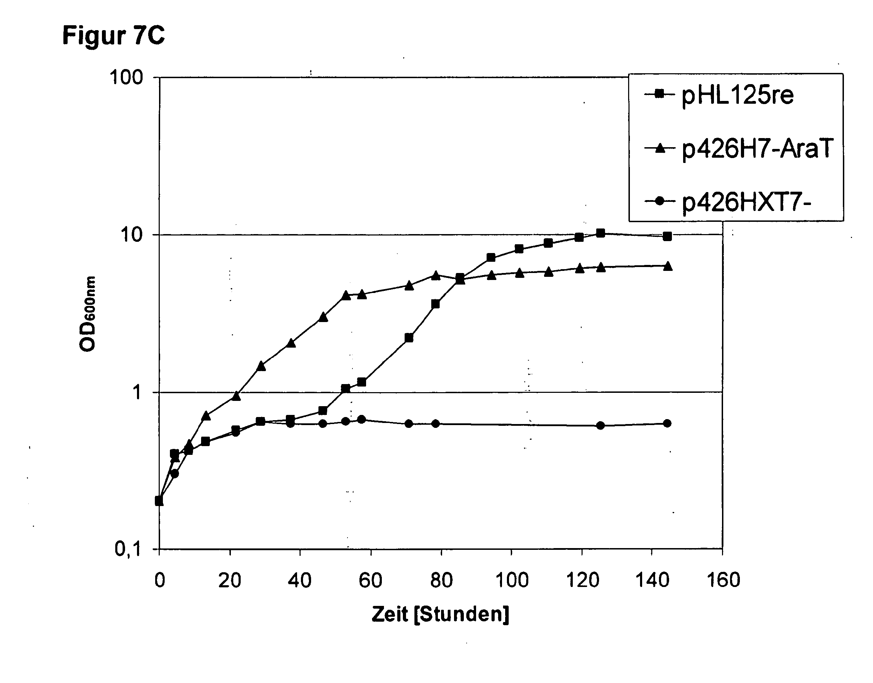

**Figur 7****.** *Wachstum auf Arabinose unter Verwendung eines spezifischen Arabinose-Transporters.*
   Wachstum des MKY06-3P, welcher zusätzlich noch das Plasmid pHL125^{re} oder das Plasmid p426H7-AraT (=MKY06-4P) enthält, in SM-Medium mit A) 0,5%, B) 1% und C) 2% L-Arabinose unter aeroben Bedingungen. Die Stämme mit den verschiedenen Plasmiden wurden in SM-Medium mit 1% L-Arabinose herangezogen und mit einer OD₆₀₀ₙₘ = 0,2 in 30ml SM-Medium mit A) 0,5%, B) 1% und C) 2% L-Arabinose angeimpft. Die Inkubation erfolgte in 300 ml Schüttelkolben unter aeroben Bedingungen bei 30°C. Mehrmals am Tag wurden Proben zur Bestimmung der optischen Dichte genommen.
**Figur 8****.** *Ethanolbildung unter Verwendung eines spezifischen Arabinose-Transporters.* Gezeigt werden die Ergebnisse von HPLC-Analysen des Stammes BWY1 mit den Plasmiden p423H7araABs^{re}, p424H7araB^{re}, p425H7araD^{re} und p426H7-AraT in SFM-Medium mit 1,7% L-Arabinose unter semi-anaeroben Bedingungen.

### Beispiele

### Methoden

### 1. Stämme und Medien

### - Bakterien

*E. coli* SURE (Stratagene)

Vollmedium LB 1% Trypton, 0,5% Hefeextrakt, 0,5% NaCl, pH 7,5 (siehe Maniatis, 1982) Für die Selektion auf eine plasmidkodierte Antibiotikaresistenz wurde dem Medium nach dem Autoklavieren 40 µg/ml Ampicillin zugesetzt. Feste Nährmedien enthielten zusätzlich 1,9% Agar. Die Anzucht erfolgte bei 37°C.

### - Hefe

### Stamm EBY.VW4000

EBY.VW4000 (Genotyp: *MATa leu2-3,112 ura3-52 trpl-289 his3-Δ1 MAL2-8c SUC2 Δhxt1-17 Δgal2 stl1Δ::loxP agt1Δ::loxP mph2Δ::loxP mph3Δ::loxP)* (Wieczorke *et al.,* 1999)

### Stamm MKY06

MKY06 (Genotyp: *MATa leu2-3,112 ura3-52 trpl-289 his3-1 MAL2-8c SUC2 hxtl-17 gal2 stl1::loxP agt1::loxP mph2::loxP mph3::loxP PromTAL1::loxP-Prom-vkHXT7,* Beschreibung: EBY.VW4000 *PromTAL1::loxP-Prom-vkHXT7)*

### Stamm MKY06-3P

MKY06-3P (Genotyp: *MATa leu2-3,112 ura3-52 trpl -289 his3-1 MAL2-8c SUC2 hxtl -17 gal2 stl1::loxP agt1::loxP mph2::loxP mph3::loxP PromTAL1::loxP-Prom-vkHXT7,* Beschreibung: EBY.VW4000 *PromTAL1::loxP-Prom-vkHXT7);* beinhaltet die Plasmide p423H7araABs", p424H7araB^{re} und p425H7araD^{re}.

### Stamm mit der Hinterlegungsnummer DSM 18544

MKY06-4P (Genotyp: *MATa leu2-3,112 ura3-52 trp1-289 his3-1 MAL2-8c SUC2 hxtl-17 gal2 stl1::loxP agt1::loxP mph2::loxP mph3::loxP PromTAL1::loxP-Prom-vkHXT7,* Beschreibung: EBY.VW4000 *PromTAL1::loxP-Prom-vkHXT7);.* beinhaltet die Plasmide p423H7araABs^{re}, p424H7araB^{re}, p425H7araD^{re} und p426H7-AraT.

### Stamm BWY1:

BWY1 basiert auf dem Stamm JBY25 (Genotyp: *MATa leu2-3,112 ura3-52 trpl-289 his3-Δ1MAL2-8c SUC2 + unbekannte Mutationen für besseres Wachstum auf L-Arabinose)* (Becker und Boles, 2003); der Stamm JBY25 wurde weiter selektioniert und besitzt noch weitere Mutationen für verbessertes Wachstum auf L-Arabinose unter Sauerstoff-limitierten Bedingungen (Wiedemann, 2005).
   - Vollmedium YEP
      1% Hefeextrakt, 2% bakteriologisches Pepton, Kohlenstoffquelle in der jeweils angegebenen Konzentration
   - synthetisches Komplettselektivmedium SC
      0,67% Yeast nitrogen base w/o amino acids and ammonium sulphate, 0,5% Ammoniumsulfat, 20mM Kaliumdihydrogenphosphat, pH 6,3, Aminosäure/Nukleobase-Lösung ohne die für die Auxotrophie-Marker der verwendeten Plasmide entsprechenden Aminosäuren, Kohlenstoffquelle in der jeweils angegeben Konzentration
   - synthetisches Minimalselektivmedium SM:
      0,67% Yeast nitrogen base w/o amino acids and ammonium sulphate, 0,5% Ammoniumsulfat, 20mM Kalium-dihydrogenphosphat, pH 6,3, Kohlenstoffquelle in der jeweils angegeben Konzentration
   - synthetisches Fermentationsmedium (Mineralmedium) SFM
      (Verduyn *et al.,* 1992), pH5,0
      Salze: (NH₄)₂SO₄, 5 g/l; KH₂PO₄, 3 g/l; MgSO₄ *7H₂O, 0,5g/l
      Spurenmetalle: EDTA, 15 mg/l; ZnSO₄ *7H₂O, 4,5 mg/l; MnCl₂ *4H₂O, 0,1mg/l;
      CoCl₂ *6H₂O, 0,3 mg/l; CuSO₄, 0,192 mg/l; Na₂MoO4 *2H₂O, 0,4 mg/l; CaCl₂
      *2H₂O, 4,5 mg/l; FeSO₄*7H₂O, 3mg/l; H₃BO₃, 1 mg/l; KI, 0,1mg/l
      Vitamine: Biotin, 0,05 mg/l; p-Aminobenzoesäure, 0,2mg/l; Nicotinsäure, 1mg/l; Calciumpantothenat, 1 mg/l; Pyridoxin-HCl, 1 mg/l; Thiamin-HCl, 1 mg/l; minositol, 25 mg/l

Konzentration der Aminosäuren und Nukleobasen im synthetischen Komplettmedium (Zimmermann, 1975): Adenin (0,08 mM), Arginin (0,22 mM), Histidin (0,25 mM), Isoleucin (0,44 mM), Leucin (0,44 mM), Lysin (0,35 mM), Methionin (0,26 mM), Phenylalanin (0,29 mM), Threonin (0,48 mM), Tryptophan (0,19 mM), Tyrosin (0,34 mM), Uracil (0,44 mM) und Valin (0,49 mM). Als Kohlenstoffquellen wurden L-Arabinose, D-Glucose, D-Galactose, D-Mannose und Maltose verwendet. Zur Selektion auf Verlust eines Plasmids mit *URA3-*Selektionsmarkergen wurden synthetische Komplettmediumsplatten verwendet, die neben Uracil 1mg/ml 5-FOA enthielten, welches nach dem Autoklavieren zugegeben wurde (Boeke *et al.,* 1984).

Feste Voll- und Selektivmedien enthielten zusätzlich 1,9% Agar. Die Anzucht der Hefezellen erfolgte bei 30°C. Das für die Fermentationen eingesetzte synthetische Mineralmedium enthielt Salze, Spurenmetalle und Vitamine in den oben aufgelisteten Konzentrationen und die angegebene Kohlenstoffquelle. Von den Spurenmetallen und den Vitaminen wurde eine Stammlösung angesetzt. Die Spurenmetalllösung wurde autoklaviert (20min, 121°C) und die Vitaminlösung sterilfiltriert. Beide wurden bei 4°C gelagert. Für die Herstellung der Spurenmetalllösung war der pH-Wert von entscheidender Rolle und verhinderte das Ausfallen einzelner Komponenten. Die verschiedenen Spurenmetalle mussten in der obigen Reihenfolge nacheinander in Wasser vollständig gelöst werden. Nach jeder Zugabe musste der pH-Wert mit KOH auf 6,0 justiert werden bevor das nächste Spurenmetall zugegeben werden konnte. Am Ende wurde der pH Wert mit HCl auf 4,0 justiert. Um Schaumbildung zu vermeiden, wurde dem Medium 50µl/l Antischaum (Antifoam204, Sigma) zugegeben. Bei anaeroben Versuchen wurde dem Medium noch 2,5ml/l einer Tween80-Ergosterol-Lösung nach dem Autoklavieren zugegeben. Diese besteht aus 16,8g Tween80 und 0,4g Ergosterol, welche mit Ethanol auf 50ml aufgefüllt und darin gelöst wurden. Die Lösung wurde sterilfiltriert. Die Salze, die entsprechenden Mengen an Spurenmetalllösung und der Antischaum wurden gemeinsam mit dem kompletten Fermenter autoklaviert. Die Kohlenstoffquelle wurde getrennt vom restlichen Medium autoklaviert. Vor dem Autoklavieren wurde bei allem der pH auf 5,0 eingestellt. Die sterile Vitaminlösung wurde nach dem Abkühlen zum Medium gegeben.

### 2. Plasmide

| Plasmid | Quelle/Referenz | Beschreibung |
|---|---|---|
| p423H7araABs^{re} | Becker und Boles, 2003 | *B. subtilis araA* in p423HXT7-His, reisoliert aus JBY25-4M |
| p424H7araB^{re} | Becker und Boles, 2003 | *E. coli araB* in p423HXT7-His; reisoliert aus JBY25-4M, Mutation in *araB* |
| p425H7araD^{re} | Becker und Boles, 2003 | *E. coli araD* in p425HXT7-His; reisoliert aus JBY25-4M |
| p426HXT7-6HIS | Becker und Boles, 2003 | 2µ Plasmid zur Überexpression von Genen und zur Herstellung von Fusionsproteinen mit 6xHis-Epitop; *URA3*-Markergen, verkürzter *HXT7*- Promotor und *CYC1*-Terminator |
| pHL125^{re} | Guldener *et al.,* 1996 | 2µ Plasmid mit dem *GAL2*-Gen exprimiert hinter dem *ADH1*-Promotor, *URA3*-Markergen, reisoliert aus JBY25-4M |
| p426H7-araT | | 2µ Plasmid mit dem *Pichia stipitis ARAT* exprimiert hinter dem verkürten *HXT7*-Promotor, *URA3-*Markergen |

### 3. Pichia stipitis Genbank

YEpTW *Pichia stipitis:* Genbank mit chromosomalen Fragmenten von *Pichia stipitis* im Überexpressionsplasmid YEp24, *URA3*-Markergen (Weierstall *et al.,* 1999)

### 4. Transformation

### - Transformation von S. cerevisiae

Die Transformation von *S*. *cerevisiae* wurde mit der Lithium-Acetat Methode (Gietz und Woods, 2002) durchgeführt. Zur Selektion auf eine Geneticin-Resistenz wurden die Zellen nach der Transformation für 4h bei 30°C in Vollmedium inkubiert und im Anschluss auf G418-haltigen Mediumsplatten ausplattiert.

### - Transformation von E. coli

Die Transformation der *E*. *coli* Zellen erfolgte durch die Elektroporationsmethode (Dower *et al.,* 1988; Wirth, 1993) mittels eines Easyject prima Geräts von EQUIBO.

### 5. Präparation von DNA

### - Isolierung von Plasmid-DNA aus S. cerevisiae

Die Zellen einer stationären Hefekultur (5 ml) wurden geerntet, gewaschen und in 100 µl Puffer 1 (entnommen aus dem "Plasmid Mini Kit") resuspendiert. Nach Zugabe von 200 µl Puffer 2 und 2/3 Volumen Glasperlen (Ø = 0,45 mm) wurden die Zellen 8 min lang auf einem Vibrax (Janke und Kunkel, Vibrax-VXR) bei 4°C aufgeschlossen. Der Überstand wurde mit 150µl Puffer 3 vermischt und für 10 min auf Eis inkubiert. Nach einer 15-minütigen Zentrifugation bei 10000 U/min wurde der Überstand verwendet und die Plasmid-DNA mit 400µl Isopropanol gefällt (-20°C, 10min). Die durch Zentrifugation (30min, 13000rpm) pelletierte DNA wurde mit 70%igem kaltem Ethanol gewaschen und in 20µl Wasser aufgenommen. Die DNA wurde anschließend für eine Transformation in *E*. *coli* oder eine DNA-Amplifizierung mittels PCR eingesetzt.

### - Isolierung von Plasmid-DNA aus E. coli

Die Isolierung von Plasmid-DNA aus *E*. coli erfolgte mit dem "Plasmid Mini Kit" der Firma Qiagen nach Angaben des Herstellers.

### - Bestimmung der DNA-Konzentration

Die DNA-Konzentration wird spektralphotometrisch in einem Wellenlängenbereich von 240-300 nm gemessen. Liegt die Reinheit der DNA, bestimmt durch den Quotient E260nm/E280nm bei 1,8, so entspricht die Extinktion E260nm = 1,0 einer DNAKonzentration von 50 µg dsDNA/ml (Maniatis, 1982).

### 6. DNA-Amplifizierung mittels PCR

### - Verwendung des Expand™ High Fidelity Systems

Die Polymerasekettenreaktion (PCR) erfolgte mit dem "Expand™ High Fidelity PCR System" der Firma Roche nach Angaben des Herstellers. Zu der zu amplifizierenden Plasmid- oder Genomischen-DNA wurden 0,2 mM dNTP-Mix, 1x Puffer 2 (enthält 1,5 mM MgCl2), 1 U Polymerase und je 100 pmol der entsprechenden Oligonukleotidprimer zusammengegeben. Die PCR-Reaktion wurde in einem Thermocycler (Techne) oder Mastercycler (Eppendorf) durchgeführt.

Zur Amplifizierung der DNA wurden folgende Temperaturzyklus gewählt.
1. 1x 95°C, 4min Denaturierung der DNA
2.18-35x 95°C, 45-60sec Denaturierung der DNA
   55-60°C, 45-60sec Bindung der Primer an die DNA (Annealing)
   72°C, 1-3min DNA Synthese (Elongation)
3. 1x 72°C, 4min DNA Synthese (Elongation)

Nach dem ersten Schritt wurde die Polymerase zugegeben ("hot start PCR"). Die Anzahl der Syntheseschritte, die Annealingtemperatur und die Elongationszeit wurden an die spezifischen Schmelztemperaturen der verwendeten Oligonukleotide bzw. an die Größe des zu erwarteten Produkts angepasst. Die PCR-Produkte wurden durch eine anschließende Agarosegelelektrophorese überprüft und anschließend aufgereinigt.

### - DNA-Aufreinigung von PCR-Produkten

Die Aufreinigung der PCR-Produkte erfolgte mit dem "QIAquick PCR Purification Kit" der Firma Qiagen nach Herstellerangaben.

### - Gelelektrophoretische Auftrennung von DNA-Fragmenten

Die Auftrennung von DNA-Fragmenten mit einer Größe von 0,15-20 kb erfolgte in 1-4%igen Agarosegelen. Als Gel- und Laufpuffer wurde 1xTAE-Puffer (40 mM Tris, 40 mM Essigsäure, 2 mM EDTA) verwendet (Maniatis, 1982). Als Größenstandard diente entweder eine mit den Restriktionsendonukleasen EcoRI und HindIII geschnittene Lambda-Phagen-DNA oder der 2-Log DNA Ladder (NEB). Die DNA-Proben wurden vor dem Auftragen mit 1/10 Volumen Blaumarker (1xTAE-Puffer, 10% Glycerin, 0,004% Bromphenolblau) versetzt. Nach der Auftrennung wurden die Gele in einem Ethidiumbromidbad inkubiert und die DNA-Fragmente durch Bestrahlung mit UV-Licht (254 nm) sichtbar gemacht.

### - Isolierung von DNA-Fragmenten aus Agarosegelen

Das gewünschte DNA-Fragment wurde aus dem TAE-Agarosegel unter langwelligem UV-Licht (366nm) ausgeschnitten und mit dem "QIAex II Gel Extraction Kit" oder dem "QIAquick Gel Extraction Kit" der Firma Qiagen nach Angaben des Herstellers isoliert.

### 7. Enzymatische Modifikation von DNA

### - DNA-Restriktion

Sequenzspezifische Spaltungen der DNA mit Restriktionsendonukleasen wurden unter den vom Hersteller empfohlenen Inkubationsbedingungen für 2-3 Stunden mit 2-5U Enzym pro µg DNA durchgerührt.

### 8. HPLC-Analysen

Die bei den Versuchen entnommenen Proben wurden für 10min bei 3000 U/min zentrifugiert, um die Hefezellen zu pellettieren. Der Überstand wurde abgenommen und sofort bei -20°C eingefroren. Für die Proteinfällung wurde später 50%ige Sulfosalizylsäure zugegeben, gemischt und für 30min bei 13000 U/min und 4°C abzentrifugiert. Der Überstand wurde abgenommen, daraus eine 1/10 Verdünnung mit Wasser hergestellt und für die HPLC-Analysen eingesetzt. Als Standards für die Messungen dienten Proben mit D-Glucose, L-Arabinose und Ethanol, welche in Konzentrationen von 0,1 %w/w, 0,5%w/w und 1,0%w/w eingesetzt wurden. Die Zucker- und die Ethanolkonzentration wurden mittels BioLC (Dionex) gemessen. Es wurde der Autosampler "AS50", der Säulenofen "TCC-100", die Gradienten-Pumpe "GS50" (alle Dionex) und der RI-Detektor "RI-101" (Shodex) bei der Messung verwendet. Als Säule wurde die VA 300/7.7 Nucleogel Sugar 810H (Macherey-Nagel) mit 20%iger Schwefelsäure als Laufmittel (0,6ml/min) benutzt. Zur Auswertung der Analysendaten wurde das Programm Chromeleon™ (Version 6.50, Dionex) verwendet.

### Beispiel 1: Aufbau eines Testsystems zur Untersuchung von L-Arabinose-Transportern

### A) Konstruktion des MKY06

In dem Hefestamm EBY.VW4000 wurden alle Gene der Hexosetransporter-Familie und zusätzlich drei Gene der Maltosetransporter-Familie deletiert. Dieser Stamm wuchs auf Maltose-Medium unverändert, konnte jedoch nicht mehr auf Glucose, Fructose und Mannose und nur sehr schwach auf Galactose wachsen (Wieczorke *et al.,* 1999). Da alle Hexosetransporter deletiert sind, ist davon auszugehen, dass der Stamm auch keine L-Arabinose mehr aufnehmen kann und sich somit zu L-Arabinose-Transportuntersuchungen eignet.

In voraus gegangenen Versuchen (siehe Becker und Boles, 2003) hatte sich gezeigt, dass zusätzlich zu einem funktionellen L-Arabinose-Stoffwechselweg auch eine erhöhte Aktivität der Transaldolase für die Verwertung von L-Arabinose nötig war. Aus diesem Grund wurde durch Austausch des endogenen Promotors von *TAL1* im EBY.VW4000 gegen den verkürzten HX*T7*-Promotor *TAL1* überexprimiert. Dieser Stamm wurde MKY06 genannt und ist dazu vorgesehen, mit den Plasmiden für den L-Arabinose-Stoffwechsel und einem Transporter, welcher L-Arabinose transportieren kann, auf dieser Kohlenstoffquelle zu wachsen.

### B) Einführung des L-Arabinose-Stoffwechselwegs

Der Stamm MKY06 wurde mit den Plasmiden p423H7araABs^{re}, p424H7araB^{re} und p425H7araD^{re} transformiert (=MKY06-3P), damit er die Fähigkeit der L-Arabinose-Verwertung erhielt. Die Transformation mit den drei Plasmiden erfolgte gleichzeitig. Die Transformanten wurden auf SC-Medium mit 2% Maltose ausplattiert. In einer weiteren Transformation wurden als Positivkontrolle noch der als L-Arabinose bekannte Transporter Gal2 und als Negativkontrolle das Leerplasmid p426HXT7-6HIS hinein transformiert und erneut auf Maltose-haltigen Mediumsplatten ausplattiert. Die Positivkontrolle, die einen L-Arabinose-Transporter und die drei Plasmide für die L-Arabinose-Verwertung enthält und die Transaldolase überexprimiert, sollte auf L-Arabinose wachsen können. Die Negativkontrolle sollte aufgrund des fehlenden Transporters kein Wachstum zeigen. Dies wurde untersucht.

### C) Überprüfung des Testsystems

Um das konstruierte Testsystem weiter verwenden zu können, mussten zuerst die Positiv- und Negativkontrolle auf ihr Wachstum hin untersucht werden. Mehrere Kolonien von den auf den SC-Platten mit 2% Maltose erhaltenen Transformanten wurden mit einer sterilen Impföse abgenommen und auf SC-Platten mit 2% L-Arabinose ausgestrichen und bei 30°C für zehn Tage inkubiert. Die Positivkontrolle zeigte nach dieser Zeit ein deutliches Wachstum und die Negativkontrolle wie erwartet kein Wachstum.

Das Wachstumsverhalten wurde ebenfalls in Flüssigmedium mit 2% Maltose oder 2% L-Arabinose untersucht. Hierzu wurden Vorkulturen mit den entsprechenden Kohlenstoffquellen (Maltose oder L-Arabinose) unter aeroben Bedingungen bei 30°C angezogen. Nach Erreichen der späten exponentiellen Phase wurden diese Vorkulturen verwendet, um 30ml des gleichen Mediums mit einer Anfangs-OD₆₀₀ₙₘ=0,2 anzuimpfen. Da die Negativkontrolle nicht auf L-Arabinose- haltigen Mediumplatten wuchs, wurde von der Vorkultur mit 2% Maltose ausgehend 30ml SC-Medium mit 2% L-Arabinose angeimpft. Das Wachstumsverhalten wurde über mehrere Tage durch Messung der optischen Dichte bei 600nm verfolgt. In Maltose-Medium zeigte die Positiv- und die Negativkontrolle wie erwartet identisches Wachstum mit einer Wachstumsrate von 0,197h⁻¹. In L-Arabinose-Wachstum zeigte sich genau wie schon bei den Versuchen mit den L-Arabinose-Mediumsplatten nur bei der Positivkontrolle Wachstum (µ = 0,01h⁻¹), welche gegenüber der Negativkontrolle noch den Transporter Gal2 besaß. Das geringe Wachstum zu Beginn bei der Negativkontrolle kann dadurch erklärt werden, dass die Zellen vor dem Umimpfen von Maltose auf L-Arabinose-haltigem Medium nicht gewaschen wurden. Außerdem ermöglichen die Glykogen-Vorräte der Hefe noch ein geringfügiges Wachstum.

Somit war dieses Testsystem funktionell und konnte für die Untersuchung von L-Arabinose-Transportern eingesetzt werden. Als Vergleich diente hierbei immer die hier genannte Positiv- und Negativkontrolle.

### Beispiel 2 Screen mit einer Pichia stipitis Genbank

Das Testsystem wurde nun verwendet, um in einer Genbank von *Pichia stipitis,* eine der Hefen die L-Arabinose verwerten können, nach möglichen L-Arabinose-Transportergenen zu suchen.

### A) Durchführung des Screens

Die hier verwendete Genbank YEpTW wurde aus dem *Pichia stipitis* Stamm CBS5774 hergestellt. Chromosomale DNA wurde mit der Restriktionsendonuklease *Sau3A* partiell verdaut und in den mit *BamHI* linearisierten Vektor YEp24 ligiert (Weierstall *et al.,* 1999).

Die Genbank besaß genau wie das Plasmid pHL125^{re} einen Uracil-Auxotrophie-Marker. Die Genbank YEpTW wurde in den Stamm MKY06-3P hinein transformiert und auf SC-Medium mit 2% Maltose ausgestrichen. Die nach drei Tagen bei 30°C erhaltenen Kolonien wurden auf SC-Mediumsplatten mit 2% L-Arabinose replikaplattiert. Nach 10 Tagen wurde nach Kolonien gesucht, die auf L-Arabinose Wachstum zeigten. Wachstum war nur möglich, wenn das Plasmid der Genbank einen Transporter kodierte, der L-Arabinose transportieren konnte.

Um genomische Mutationen, welche für das Wachstum verantwortlich sein könnten, ausschließen zu können, wurden die gefundenen Kolonien auf Komplettmedium mit 5-FOA ausgestrichen. Dadurch wurde auf einen Verlust des Plasmids der Genbank selektioniert. Diese Kolonien der 5-FOA Platte konnten bei erneutem Ausstreichen auf L-Arabinose-Medium hierauf nicht mehr wachsen. Somit konnte eine genomische Mutation als Ursache für das Wachstum ausgeschlossen werden. Die gefundenen Kolonien wurden auch noch auf anderen Kohlenstoffquellen ausgestrichen, um das Substratspektrum auszutesten.

### B) Wachstumsverhalten

Von den über 30000 replikaplattierten Kolonien wurden die elf hierbei gefundenen Kolonien, welche geringfügiges Wachstum zeigten, nochmals auf L-Arabinose-Platten ausgestrichen. Hierbei zeigten aber nur fünf Kolonien erneut Wachstum auf L-Arabinose. Es handelte sich hierbei um den Stamm MKY06-3P, welcher zusätzlich noch das Plasmid pAra1, pAraT6, pAraT7, pAraT8 oder pAraT11 aus der Genbank YEpTW (siehe Figur 4) enthielt. Das stärkste Wachstum zeigte der MKY06-3P mit dem AraT11. Die restlichen wuchsen im Vergleich mit der Positivkontrolle schwächer. Beachten muss man hierbei aber, dass das *GAL2* in der Positivkontrolle durch einen starken Promotor überexprimiert wurde und die Gene auf den Plasmiden der Genbank ihren nativen Promotor besaßen. Von besonderem Interesse sind die Plasmide pAraT1 und pAraT7, da diese nur auf L-Arabinose-Medium Wachstum vermittelten. Diese zeigten auf D-Glucose und auf D-Mannose kein Wachstum. Auf D-Galactose bewirkte lediglich das pAraT1 geringfügiges Wachstum. Das Wachstum des pAraT7 auf D-Galactose entsprach dem schwachen Wachstum der Negativkontrolle (vgl. Figur 4). Dieses wurde schon früher für den Ausgangsstamm des MKY06, den EBY.VW4000, berichtet (Wieczorke *et al.,* 1999).

Die Kolonien mit dem pAraT6, pAraT7 und pAraT11 wurden in flüssigem SC-Medium mit 2% L-Arabinose herangezogen und hiervon Glycerinkulturen angefertigt, welche für spätere. Untersuchungen bei -70°C gelagert werden. Mit den Plasmiden pAraT1 und pAraT7 wurde weiter gearbeitet und das Wachstumsverhalten der Stämme in Flüssigmedium untersucht. In SC-Medium mit 2% Maltose verhielt sich der MKY06-3P, welcher zusätzlich das pAraT1 oder pAraT7 enthielt, identisch zu der Positiv- (zusätzlich noch pHL125^{re}) und der Negativkontrolle (zusätzlich noch p426HXT7-6HIS). Die Wachstumsraten betrugen 0,197h⁻¹.

Auf SC-Medium mit 2% L-Arabinose zeigten sich Unterschiede. Die Negativkontrolle (p426HXT7-6HIS) zeigte kein Wachstum auf diesem Medium. Das geringe Wachstum zu Beginn resultierte von dem fehlenden Waschschritt bei der Umimpfung von der Vorkultur auf Maltose-Medium zu der Hauptkultur in L-Arabinose-Medium. Beide gefundenen L-Arabinose-Transporter verhielten sich ähnlich. In der Wachstumsrate (µ = 0,087h⁻¹) als auch in der maximalen OD₆₀₀ₙₘ gab es keine Unterschiede zwischen dem MKY06-3P mit dem Plasmid pAraT1 oder dem pAraT7. Vergleicht man das Wachstumsverhalten der zwei neuen Transporter mit der Positivkontrolle (pHL125^{re}, µ = 0,1h⁻¹) so zeigte sich eine etwas niedrigere Wachstumsrate und eine niedrigere Maximale OD₆₀₀ₙₘ.

### C) Isolierung der gefundenen L-Arabinose Transporter

Um die gefundenen Transporter auf genomischer Ebene analysieren zu können, mussten zuerst die Genbank-Plasmide wieder aus der Hefe isoliert werden. Hierbei war zu beachten, dass der Stamm MKY06 nicht nur das Plasmid aus der Genbank YEpTW enthält, welches den gesuchten Transporter kodiert, sondern gleichzeitig auch noch die drei Plasmide für den L-Arabinose-Stoffwechsel (p423H7araABs^{re}, p424H7araB^{re}, p425H7araD^{re}) und dass das Plasmid p423H7araABs" in einer viel höheren Anzahl in den Zellen vorkommt, als die restlichen drei Plasmide. Da die Plasmide der Genbank YEpTW einen Uracil-Auxotrophie-Marker besaßen, wurden die Zellen von den L-Arabinose-Platten ausgehend in Maltose-Flüssigmedium ohne Uracil (ura-) angeimpft. Nach Erreichen der stationären Phase wurden diese in frisches Maltose-ura-Medium umgeimpft und wieder herangezogen. Dabei wurden die Zellen mit den vier Plasmiden fünfmal in Maltose-ura- Flüssigmedium bis zur stationären Phase herangezogen. Ziel hiervon war eine Anreicherung des Plasmids pAraT1 beziehungsweise pAraT7. Von diesen beiden Kulturen wurden Vereinzelungsausstriche auf Maltose-ura- Mediumsplatten angefertigt. Die entstandenen Kolonien wurden auf weitere Maltose Platten replikaplattiert und für zwei Tage bei 30°C inkubiert. Diese Platten enthielten die entsprechende Aminosäure für den Auxotrophie-Marker eines der anderen drei Plasmide (Histidin für p423H7araABs^{re}, Tryptophan für p424H7araB^{re} oder Leucin für p425H7araD^{re}) nicht. Diese Replikaplatten wurden mit den Maltose-ura- Platten verglichen. Kolonien, die nur auf der Maltose-ura- Platte wuchsen, wurden ausgewählt. Diese besaßen nur noch das Plasmid pAraT1 beziehungsweise pAraT7. Die Plasmide wurden aus Hefe isoliert. Danach wurden die Plasmide in *E*. *coli* amplifiziert, und nach der Isolierung aus *E*. *coli* mit Hilfe einer Restriktionsanalyse charakterisiert. Als Restriktionsenzyme wurden NcoI und NheI verwendet. NcoI schneidet nur im URA3-Markergen. Wenn es sich um das gesuchte Plasmid aus der Genbank handelt, dann entsteht ein 934bp großes Fragment.

### Beispiel 3 Charakterisierung des neuen Arabinose-Transporters (araT)

### A) Sequenzierung

Die auf den in Beispiel 2 gefundenen Plasmiden pAraT1 und pAraT7 lokalisierten chromosomalen Fragmente aus *P*. *stipitis* wurden sequenziert.

Der komplette ORF des gefundenen Transporters wurde doppelsträngig mit überlappenden Bereichen sequenziert. Der Promotor und Terminator-Bereich wurde einzelsträngig sequenziert (vgl. Figur 5). Die Pfeile zeigen die Bereiche von einzelnen Sequenzierungen an.

Bei der Sequenzierung zeigte sich, daß die zwei Plasmide pAraT1 und pAraT7 überlappende Fragmente des gleichen Gens beinhalten. Es handelt sich um ein und denselben Transporter und nicht um zwei verschiedene Transportergene. Das Plasmid pAraT1 besitzt ein Insert mit etwa 5kb; es beinhaltet den kompletten offenen Leserahmen (ORF) des AraT, welcher aus 1629 Basen und folglich 542 Aminosäuren (plus das STOP-Codon) besteht. Zusätzlich noch Promotor und Terminator-Sequenzen. Das Plasmid pAraT7 besitzt ein Insert, welches etwa 3kb groß ist; es beinhaltet nicht den kompletten ORF des AraT, sondern nur die ersten 1507 Basen. Dieses Fragment war aber trotzdem funktionell.

Bei einer BLAST-Recherche mit dem kürzlich veröffentlichten Genom von *Pichia stipitis* (siehe http://genome.jgi-psf.org/euk_home.html) zeigte sich eine Übereinstimmung von 100 % mit HGT2. HGT2 wurde aufgrund seiner hohen Homologie zum hochaffinen Glucose-Transporter HGT1 von *Candida albicans* als putativer hochaffiner Glucose-Transporter annotiert. Wenn man die Sequenz hinsichtlich der möglichen Transmembran-Domänen untersucht, erhält man 12 Transmembran-Domänen, was für Transporter typisch ist.

### B) Beispiele für Vektoren für der AraT

Ausgangsplasmid für die Herstellung der Genbank war das Plasmid YEp24. Das Plasmid pAraT1 als auch das Plasmid pAraT7 basieren somit auf dem YEp24 und unterscheiden sich nur in der Größe des Inserts. Bei dem Vektor YEp24 handelt es sich um ein episomales Plasmid.

Der offene Leserahmen (ORF) des gefundenen Arabinose-Transporters wurde von dem pAraT1 amplifiziert und hinter den verkürzten starken *HXT7*-Promotor des Plasmids p426HXT7-6HIS kloniert. Hierbei entstand das Plasmid p426H7-AraT, welches einen Uracil-Marker besitzt.

Ein anderes mögliches Expressionsplasmid ist p426Met25. Für Vektorkarten, siehe Figuren 6A bis 6E.

Weitere mögliche Expressionsvektoren sind pYES260, pYES263, pVTU260, pVTU263, pVTL260 und pVTL263. Nähere Angaben zu diesen Vektoren finden sich unter http://web.uni-frankfurt.de/fb15/mikro/euroscarf/data/km_expr.html.

### C) Wachstum in Abhängigkeit von der L-Arabinose-Konzentration im Medium

Das Wachstum des Stammes MKY06-4P wurde unter aeroben Bedingungen in Abhängigkeit von der L-Arabinose-Konzentration im Medium untersucht. Als Vergleich wurde der Stamm MKY06-3P verwendet, der zusätzlich noch das Plasmid pHL125^{re} oder p426HXT7-6HIS enthielt.

Die Stämme mit den verschiedenen Plasmiden wurden in SM-Medium mit 1 % L-Arabinose herangezogen und mit einer OD₆₀₀ₙₘ = 0,2 in 30ml SM-Medium mit 0,5%, 1% bzw. 2% L-Arabinose angeimpft. Die Inkubation erfolgte in 300ml Schüttelkolben unter aeroben Bedingungen bei 30°C. Mehrmals am Tag wurden Proben zur Bestimmung der optischen Dichte genommen.

Die Ergebnisse sind in den Figuren 7A bis 7C gezeigt. Der Stamm MKY06-4P wächst unter allen 3 Bedingungen schneller als der Vergleichsstamm MKY06-3P; welcher noch das Plasmid pHL125^{re} enthält. Es zeigt sich bei 0,5% L-Arabinose (Figur 7A) ein deutlicher Vorteil des p426H7-AraT gegenüber des pHL125^{re}. Der Stamm wächst deutlich schneller und auch zu einer höheren optischen Dichte heran. Auch bei 1% L-Arabinose (Figur 7B) wächst der Stamm mit dem p426H7-AraT schneller als der Vergleichsstamm. In der am Ende des Wachstums erreichten optischen Dichte zeigt sich jedoch kein Unterschied. Auch bei einer L-Arabinose Konzentration von 2% (Figur 7C) wächst der Stamm mit dem p426H7-AraT schneller als der Vergleichsstamm mit dem pHL125^{re}, welcher aber bei dieser Konzentration eine höhere optische Dichte am Ende des Wachstums erreicht.

Damit wird gezeigt, daß das erfindungsgemäße L-Arabinose-Aufnahmesystem es den rekombinanten *S*. *cerevisiae* Zellen ermöglicht, L-Arabinose wesentlich effizienter zu verwerten.

### Beispiel 4 Verwendung des neuen Arabinose-Transporters (araT) zur Bildung von Ethanol

In Figur 8 werden die Ergebnisse von HPLC-Analysen des Stammes BWY1 mit den Plasmiden p423H7araABs^{re}, p424H7araB^{re}, p425H7araD^{re} und zusätzlich dem p426H7-AraT oder als Vergleich dem pHL125^{re} in SFM-Medium mit 1,7% L-Arabinose unter semi-anaeroben Bedingungen gezeigt.

Die Stämme wurden aerob im gleichen Medium zu einer hohen optischen Dichte herangezogen. Die Zellen wurden abzentrifugiert und zum Animpfen der semi-anaeroben Fermentations

Versuche verwendet. Bereits nach etwa 25 Stunden setzt bei beiden Stämmen die Ethanol Produktion ein. In dem Stamm, der das Plasmid p426H7-AraT beinhaltet ist eine höhere Ethanol Produktion zu Beginn festzustellen. Andererseits nimmt die Arabinose Konzentration am Ende des Versuches bei Zellen mit dem p426H7-AraT stärker ab als bei dem pHL125^{re}, was darauf hindeutet, dass hier eine höhere Affinität des AraT zu einer verbesserten Fermentation von niedrigen Arabinose-Konzentrationen fühlt.

### Referenzen

Becker, J. und Boles, E. (2003) A modified Saccharomyces cerevisiae strain that consumes L-Arabinose and produces ethanol. Appl Environ Microbiol 69(7), 4144-50.
Boeke, J.D., LaCroute, F. und Fink, G.R. (1984) A positive selection for mutants lacking orotidine-5'-phosphate decarboxylase activity in yeast: 5-fluoroorotic acid resistance. Mol Gen Genet 197(2), 345-6.
Boles E. (2002) in "Transmembrane Transporters", Editor Michael W. Quick, WILEY-LISS, 19-36.
Burke, D., Dawson, D., Stearns, T. (2000) Methods in Yeast Genetics. A Cold Spring Harbor Laboratory Course Manual. Cold Spring Harbor Laboratory Press.
Dien, B.S., Kurtzman, C.P., Saha, B.C. und Bothast, R.J. (1996) Screening for L-arabinose fermenting yeasts. Appl Biochem Biotechnol 57-58, 233-42.
Dower, W.J., Miller, J.F. und Ragsdale, C.W. (1988) High efficiency transformation of E. coli by high voltage electroporation. Nucleic Acids Res 16(13), 6127-45.
Du Preez, J.C., Bosch, M. und Prior, B.A. (1986) Xylose fermentation by Candida shehatae and Pichia stipitis - effects pf pH, temperature and substrate concentration. Enzyme Microb Technol 8(360-364).
Gietz, R.D. und Woods, R.A. (2002) Transformation of yeast by lithium acetate/singlestranded carrier DNA/polyethylene glycol method. Methods Enrymol 350, 87-96. Hahn-Hagerdal, B., Wahlbom, C.F., Gardonyi, M., van Zyl, W.H., Cordero Otero, R.R. und Jonsson, L.J. (2001) Metabolic engineering of Saccharomyces cerevisiae for xylose utilization. Adv Biochem Eng Biotechnol 73, 53-84.
Jeppsson M, Bengtsson O, Franke K, Lee H, Hahn-Hagerdal B, Gorwa-Grauslund MF. (2006) The expression of a Pichia stipitis xylose reductase mutant with higher K(M) for NADPH increases ethanol production from xylose in recombinant Saccharomyces cerevisiae. Biotechnol Bioeng. 93(4):665-73.
Jin YS, Jeffries TW. (2004) Stoichiometric network constraints on xylose metabolism by recombinant Saccharomyces cerevisiae. Metab Eng. 6(3):229-38.
Katahira S, Mizuike A, Fukuda H, Kondo A. (2006) Ethanol fermentation from lignocellulosic hydrolysate by a recombinant xylose- and cellooligosaccharide-assimilating yeast strain. Appl Microbiol Biotechnol. 72(6):1136-43.
Kotter, P. und Ciriacy, M. (1993) Xylose fermentation by Sacharomyces cerevisiae. Appl Microbiol Biotechnol 38, 776-783.
Kou, S.C., Christensen, M.S. und Cirillo, V.P. (1970) Galactose transport in Saccharomyces cerevisiae. II. Characteristics of galactose uptake and exchange in galactokinaseless cells. J Bacteriol 103(3), 671-8*.*
Kuyper, M., Toirkens, M.J., Diderich, J.A., Winkler, A.A., van Dijken, J.P. und Pronk, J.T. (2005b) Evolutionary engineering of mixed-sugar utilization by a xylose-fermenting Saccharomyces cerevisiae strain. FEMS Yeast Res 5(10), 925-34.
Kuyper, M., Winkler, A.A., van Dijken, J.P. und Pronk, J.T. (2004) Minimal metabolic engineering of Saccharomyces cerevisiae for efficient anaerobic xylose fermentation: a proof of principle. FEMS Yeast Res 4(6), 655-64.
Lucas, C. und Uden, N.v. (1986) Transport of hemicellulose monomers in the xylose-fermenting yeast Candida shehatae. Appl Microbiol Biotechnol 23, 491-495.
Maniatis, T., E.F. Fritsch und J. Sambrook (1982) Molecular cloning. A laboratory manual. New York: Cold Spring Harbor.
Pitkanen JP, Rintala E, Aristidou A, Ruohonen L, Penttila M. (2005) Xylose chemostat isolates of Saccharomyces cerevisiae show altered metabolite and enzyme levels compared with xylose, glucose, and ethanol metabolism of the original strain. Appl Microbiol Biotechnol. 67(6):827-37.
Richard, P., Putkonen, M., Vaananen, R., Londesborough, J. und Penttila, M. (2002) The missing link in the fungal L-arabinose catabolic pathway, identification of the L-xylulose reductase gene. Biochemistry 41(20), 6432-7.
Verduyn, C., Postma, E., Scheffers, W.A. und Van Dijken, J.P. (1992) Effect of benzoic acid on metabolic fluxes in yeasts: a continuous-culture study on the regulation of respiration and alcoholic fermentation. Yeast 8(7), 501-17.
Weierstall, T., Hollenberg, C.P. und Boles, E. (1999) Cloning and characterization of three genes (SUT1-3) encoding glucose transporters of the yeast Pichia stipitis. Mol Microbiol 31(3),871-83.
Wieczorke, R., Krampe, S., Weierstall, T., Freidel, K., Hollenberg, C.P. und Boles, E. (1999) Concurrent knock-out of at least 20 transporter genes is required to block uptake of hexoses in Saccharomyces cerevisiae. FEBS Lett 464(3), 123-8.
Wiedemann, B. (2005) Molekulargenetische und physiologische Charakterisierung eines rekombinanten Pentose-vergärenden Hefestammes. Diplomarbeit. Johann Wolfgang Goethe-Universität, Frankfurt am Main.
Wirth, R. (1993) Elektroporation: Eine alternative Methode zur Transformation von Bakterien mit Plasmid-DNA.. Forum Mikrobiologie 11(507-515).
Zaldivar, J., Nielsen, J. und Olsson, L. (2001) Fuel ethanol production from lignocellulose: a challenge for metabolic engineering and process integration. Appl Microbiol Biotechnol 56(1-2), 17-34.
Zimmermann, F.K. (1975) Procedures used in the induction of mitotic recombination and mutation in the yeast Saccharomyces cerevisiae. Mutat Res 31(2), 71-86.

## Patentansprüche

1. Polypeptid, ausgewählt aus der Gruppe von
a. einem Polypeptid, das zu mindestens 80% identisch zu der Aminosäuresequenz nach SEQ ID NO: 1 ist und eine *in vitro* und/oder *in vivo* Pentose-Transportfunktion aufweist, und
b. einem Polypeptid, das identisch zu der Aminosäuresequenz nach SEQ ID NO:1 ist und eine *in vitro* und/oder *in vivo* Pentose-Transportfunktion aufweist,
wobei die Pentose bevorzugt Arabinose, insbesondere L-Arabinose ist.

2. Polypeptid nach Anspruch 1,
umfassend ein Fragment von mindestens 200 oder 300 kontinuierlichen Aminosäuren nach SEQ ID NO: 1, das eine *in vitro* und/oder *in vivo* Pentose-Transportfunktion aufweist, bevorzugt umfassend ein Fragment von 502 Aminosäuren, das den ersten 502 Aminosäuren nach SEQ ID NO: 1 entspricht und eine *in vitro* und/oder *in vivo* Pentose-Transportfunktion aufweist,
und/oder
wobei das Polypeptid zu mindestens 90%, bevorzugt 95% identisch zu der Aminosäuresequenz nach SEQ ID NO:1 ist und eine *in vitro* und/oder *in vivo* Pentose-Transportfunktion aufweist,
und/oder
weiter umfassend heterologe Aminosäuresequenzen,
wobei die Pentose bevorzugt Arabinose, insbesondere L-Arabinose ist.

3. Polypeptid nach Anspruch 1 oder 2, wobei das Polypeptid aus einer Hefe, bevorzugt aus *Pichia,* insbesondere *Pichia stipitis,* abstammt.

4. Isoliertes Nukleinsäuremolekül, kodierend für ein Polypeptid nach einem der Ansprüche 1 bis 3,
wobei die Nukleinsäure bevorzugt zu mindestens 90%, bevorzugt 95% und weiter bevorzugt 99% identisch zu der Nukleinsäuresequenz nach SEQ ID NO:2 oder 3 ist,
wobei das Nukleinsäuremolekül bevorzugt dsDNA, ssDNA, PNA, CNA, RNA oder mRNA oder Kombinationen davon umfaßt.

5. Nukleinsäuremolekül nach Anspruch 4, weiter umfassend Vektor-Nukleinsäuresequenzen, bevorzugt Expressionsvektor-Sequenzen,
bevorzugt weiter umfassend Nukleinsäuresequenzen, die für weitere heterologe Polypeptide kodieren,
wobei das Nukleinsäuremolekül bevorzugt dsDNA, ssDNA, PNA, CNA, RNA oder mRNA oder Kombinationen davon umfaßt.

6. Wirtszelle, die ein Nukleinsäuremolekül nach Anspruch 4 oder 5 enthält und bevorzugt dieses exprimiert,
die bevorzugt eine Pilzzelle und weiter bevorzugt eine Hefezelle, wie *Saccharomyces* species, *Kluyveromyces* sp., *Hansenula* sp., *Pichia* sp. oder *Yarrowia* sp., ist.

7. Wirtszelle nach Anspruch 6, weiterhin enthaltend Nukleinsäuremoleküle, die für Proteine des Arabinose-Stoffwechselweges kodieren,
wobei die Nukleinsäuremoleküle bevorzugt für Proteine des bakteriellen Arabinose-Stoffwechselweges kodieren.

8. Wirtszelle des Stammes MKY06-4P, der am 23. August 2006 bei der Deutschen Sammlung von Mikroorganismen und Zellkulturen unter der Hinterlegungsnummer DSM 18544 hinterlegt wurde.

9. Antikörper oder Antikörper-Fragment, umfassend einen immunologisch aktiven Teil, der selektiv an ein Polypeptid nach einem der Ansprüche 1 bis 3 bindet.

10. Verfahren zur Herstellung eines Polypeptids nach einem der Ansprüche 1 bis 3, umfassend Kultivieren der Wirtszelle nach einem der Ansprüche 6 bis 8 unter Bedingungen, unter denen das Nukleinsäuremolekül nach einem der Ansprüche 4 oder 5 exprimiert wird.

11. Verfahren zur Identifizierung einer Verbindung, die an ein Polypeptid nach einem der Ansprüche 1 bis 3 bindet und/oder seine Aktivität moduliert, umfassend die Schritte von:
In Kontakt bringen eines Polypeptids oder einer Zelle, die ein Polypeptid nach einem der Ansprüche 1 bis 3 exprimiert, mit einer Testverbindung, und
Bestimmen, ob das Polypeptid an die Testverbindung bindet und, gegebenenfalls, Bestimmen, ob die Testverbindung die Aktivität des Polypeptids moduliert,
wobei die Verbindung bevorzugt eine Pentose, wie zum Beispiel Arabinose, und insbesondere L-Arabinose, oder ein Derivat einer solchen Pentose ist.

12. Verfahren zur Modulierung der Aktivität eines Polypeptids nach einem der Ansprüche 1 bis 3, umfassend in Kontakt bringen eines Polypeptids oder einer Zelle, die ein Polypeptid nach einem der Ansprüche 1 bis 3 exprimiert, mit einer Verbindung, die an das Polypeptid bindet in einer ausreichenden Konzentration, um die Aktivität des Polypeptids zu modulieren, wobei die Verbindung bevorzugt eine Pentose, wie zum Beispiel Arabinose, und insbesondere L-Arabinose, oder ein Derivat einer solchen Pentose ist.

13. Verfahren zur Produktion von Bioethanol, umfassend die Expression eines Nukleinsäuremoleküls nach einem der Ansprüche 4 oder 5 in einer Wirtszelle nach einem der Ansprüche 6 bis 8.

14. Verwendung eines Polypeptids nach einem der Ansprüche 1 bis 3, eines Nukleinsäuremoleküls nach einem der Ansprüche 4 oder 5 oder einer Wirtszelle nach einem der Ansprüche 6 bis 8 zur Produktion von Bioethanol und/oder zur rekombinanten Fermentation von Pentose-haltigem Biomaterial.

## Claims

1. Polypeptide, selected from the group of
a. a polypeptide which is at least 80% identical to the amino acid sequence according to SEQ ID NO: 1 and has an *in vitro* and/or *in vivo* pentose transport function, and
b. a polypeptide which is identical to the amino acid sequence according to SEQ ID NO:1 and has an *in vitro* and/or *in vivo* pentose transport function
wherein the pentose is preferably arabinose, in particular L-arabinose.

2. Polypeptide according to Claim 1, comprising a fragment of at least 200 or 300 continuous amino acids according to SEQ ID NO:1. which has an *in vitro* and/or *in vivo* pentose transport function, preferably comprising a fragment of 502 amino acids, which corresponds to the first 502 amino acids according to SEQ ID NO:1. and has an *in vitro* and/or *in vivo* pentose transport function,
and/or
wherein the polypeptide is at least 90%, preferably 95% identical to the amino acid sequence according to SEQ ID NO:1 and has an *in vitro* and/or *in vivo* pentose transport function.
and/or
further comprising heterologous amino acid sequences.
wherein the pentose is preferably arabinose, in particular L-arabinose.

3. Polypeptide according to Claim 1 or 2, wherein the polypeptide originates from a yeast, preferably from *Pichia,* in particular *Pichia stipitis.*

4. Isolated nucleic acid molecule, coding for a polypeptide according to any of Claims 1 to 3,
wherein the nucleic acid is preferably at least 90%, preferably 95%, and more preferably 99% identical to the nucleic acid sequence according to SEQ ID NO:2 or 3,
wherein the nucleic acid molecule preferably comprises dsDNA, ssDNA, PNA, CNA, RNA or mRNA or combinations thereof.

5. Nucleic acid molecule according to Claim 4, further comprising vector nucleic acid sequences, preferably expression vector sequences, preferably further comprising nucleic acid sequences which code for further heterologous polypeptides,
wherein the nucleic acid molecule preferably comprises dsDNA, ssDNA, PNA, CNA, RNA or mRNA or combinations thereof.

6. Host cell, containing a nucleic acid molecule according to Claim 4 or 5 and preferably expressing said nucleic acid molecule,
wherein said host cell is preferably a fungus cell and more preferably a yeast cell, such as *Saccharomyces* species, *Kluyveromyces* sp., *Hansenula* sp., *Pichia* sp. or *Yarrowia* sp.

7. Host cell according to Claim 6, further containing nucleic acid molecules which code for proteins of the arabinose metabolic pathway, wherein the nucleic acid molecules preferably code for proteins of the bacterial arabinose metabolic pathway.

8. Host cell of the strain MKY06-4P, which was deposited on 23^{rd} August 2006 with the German Collection of Microorganisms and Cell Cultures under deposition number DSM 18544.

9. Antibody or antibody fragment, comprising an immunologically active part, which binds selectively to a polypeptide according to any of Claims 1 to 3.

10. Method for the production of a polypeptide according to any of Claims 1 to 3, comprising cultivating the host cell according to any of Claims 6 to 8 under conditions by which the nucleic acid molecule according to any of the Claims 4 or 5 is expressed.

11. Method for identifying a compound which binds to a polypeptide according to any of Claims 1 to 3 and/or modulates its activity, comprising the steps of:
contacting a polypeptide or a cell, which expresses a polypeptide according to any of Claims 1 to 3, with a test compound, and
determining whether the polypeptide binds to the test compound and,
if applicable, determining whether the test compound modulates the activity of the polypeptide,
wherein the compound is preferably a pentose, such as arabinose, and in particular L-arabinose, or a derivative of such a pentose.

12. Method for modulating the activity of a polypeptide according to any of Claims 1 to 3, comprising
contacting a polypeptide or a cell, which expresses a polypeptide according to any of Claims 1 to 3, with a compound which binds to the polypeptide in a concentration which is sufficient to modulate the activity of the polypeptide,
wherein the compound is preferably a pentose, such as arabinose, and in particular L-arabinose, or a derivative of such a pentose.

13. Method for the production of bioethanol, comprising the expression of a nucleic acid molecule according to any of Claims 4 or 5 in a host cell according to any of Claims 6 to 8

14. Use of a polypeptide according to any of Claims 1 to 3, of a nucleic acid molecule according to any of Claims 4 or 5 or of a host cell according to any of Claims 6 to 8 for the production of bioethanol and/or for the recombinant fermentation of biomaterial containing pentose.

## Revendications

1. Polypeptide, choisi dans le groupe comprenant
a. un polypeptide, qui est identique à au moins 80% à la séquence d'acide aminé selon SEQ ID NO:1 1 et qui présente une fonction *in vitro* et/ou *in vivo* de transport du pentose et
a. un polypeptide, qui est identique à la séquence d'acide aminé selon SEQ ID NO:1 et qui présente une fonction *in vitro* et/ou *in vivo* de transport du pentose,
le pentose étant de préférence de l'arabinose, notamment du L-arabinose.

2. Polypeptide selon la revendication 1,
comprenant un fragment d'au moins 200 ou 300 acides aminés continus selon la SEQ ID NO:1, qui présente une fonction *in vitro* et/ou *in vivo* de transport du pentose,
comprenant de préférence un fragment de 502 acides aminés, qui correspond aux 502 premiers acides aminés selon la SEQ ID NO:1 et qui présente une fonction *in vitro* et/ou *in vivo* de transport du pentose,
et/ou
a. le polypeptide étant identique à au moins 90%, de préférence à 95% à la séquence d'acide aminé selon la SEQ ID NO:1 et présentant une fonction *in vitro* et/ou *in vivo* de transport du pentose,
et/ou
comprenant en outre des séquences hétérologues d'acide aminé,
le pentose étant de préférence de l'arabinose, notamment du L-arabinose.

3. Polypeptide selon la revendication 1 ou 2, le polypeptide provenant d'une levure, de préférence de la *Pichia,* notamment de la *Pichia stipitis.*

4. Molécule isolée de l'acide nucléique, codante pour un polypeptide selon l'une quelconque des revendications 1 à 3,
l'acide nucléique étant identique à au moins 90%, de préférence à au moins 95%, de manière encore plus préférée, à au moins 99% à la séquence d'acide nucléique selon SEQ ID NO:2 ou 3,
la molécule d'acide nucléique comprenant de préférence du dsADN, du ssADN, de l'APN, de l'ACN, de l'ARN ou du mARN ou des associations de ces derniers.

5. Molécule de l'acide nucléique selon la revendication 4, comprenant par ailleurs des séquences d'acide nucléique vecteur, de préférence des séquences du vecteur d'expression, comprenant de préférence par ailleurs des séquences de l'acide nucléique codant pour d'autres polypeptides hétérologues,
la molécule d'acide nucléique comprenant de préférence du dsADN, du ssADN, de l'APN, de l'ACN, de l'ARN ou du mARN ou des associations de ces derniers.

6. Cellule hôte qui contient une molécule de l'acide nucléique selon la revendication 4 ou 5 et qui de préférence exprime cette dernière,
qui est de préférence une cellule fongique et encore de préférence une cellule de levure, telle que les espèces de *Saccharomyces,* de *Kluyveromyces, d'Hansenula,* de *Pichia* ou d'*Yarrowia.*

7. Cellule hôte selon la revendication 6, contenant par ailleurs des molécules de l'acide nucléique codantes pour la protéine de la voie métabolique de l'arabinose,
les molécules de l'acide nucléique codant de préférence pour la protéine de la voie métabolique bactérienne de l'arabinose.

8. Cellule hôte de la souche MKY06-4P qui a été déposée le 23 août 2006 dans la collection allemande de micro-organismes et de cultures cellulaires sous le numéro de dépôt DSM 18544.

9. Anticorps ou fragment d'anticorps, comprenant une partie immunologiquement active qui se lie de manière sélective à un polypeptide selon l'une quelconque des revendications 1 à 3.

10. Procédé de production d'un polypeptide selon l'une quelconque des revendications 1 à 3, comprenant la culture de la cellule hôte selon l'une quelconque des revendications 6 à 8 dans des conditions dans laquelle la molécule de l'acide nucléique selon l'une quelconque des revendications 4 ou 5 est exprimée.

11. Procédé d'identification d'un composé qui se lie à un polypeptide selon l'une quelconque des revendications 1 à 3 et/ou qui module son activité, comprenant les étapes de :
Mise en contact d'un polypeptide ou d'une cellule exprimant un polypeptide selon l'une quelconque des revendications 1 à 3 avec un composé test et
Détermination si le polypeptide se lie au composé test et le cas échéant,
Détermination si le composé test module l'activité du polypeptide,
le composé étant de préférence un pentose, tel que par exemple l'arabinose et notamment du L-arabinose ou un dérivé d'un tel pentose.

12. Procédé de modulation de l'activité d'un polypeptide selon l'une quelconque des revendications 1 à 3, comprenant la mise en contact d'un polypeptide ou d'une cellule exprimant un polypeptide selon l'une quelconque des revendications 1 à 3 avec un composé que se lie au polypeptide dans une concentration suffisante pour moduler l'activité du polypeptide,
le composé étant de préférence un pentose, tel que par exemple l'arabinose et notamment du L-arabinose ou un dérivé d'un tel pentose.

13. Procédé de production de bioéthanol, comprenant l'expression d'une molécule de l'acide nucléique selon l'une quelconque des revendications 4 ou 5 dans une cellule hôte selon l'une quelconque des revendications 6 à 8.

14. Utilisation d'un polypeptide selon l'une quelconque des revendications 1 à 3, d'une molécule de l'acide nucléique selon l'une quelconque des revendications 4 ou 5 ou d'une cellule hôte selon l'une quelconque des revendications 6 à 8 pour la production de bioéthanol et/ou pour la fermentation recombinante de matériel bio contenant du pentose.
